(19) Europäisches Patentamt
European Patent Office
Office européen des brevets

(11) Publication number: **0 103 465**
**B1**

(12) # EUROPEAN PATENT SPECIFICATION

(45) Date of publication of patent specification: **10.09.86**

(51) Int. Cl.⁴: **C 07 H 17/08**, A 61 K 31/70, A 23 K 1/17

(21) Application number: **83305241.8**

(22) Date of filing: **08.09.83**

(54) **20-Amino macrolide derivatives.**

(30) Priority: **13.09.82 US 417247**

(43) Date of publication of application:
**21.03.84 Bulletin 84/12**

(45) Publication of the grant of the patent:
**10.09.86 Bulletin 86/37**

(84) Designated Contracting States:
**BE CH DE FR GB IT LI LU NL SE**

(56) References cited:
**EP-A-0 087 921**

**CHEM. PHARM. BULL., vol. 30, no. 1, 1982, page 97-110, H. MATSUBARA et al.: "Chemical transformation of tylosin, 1 16-membered macrolide, and its structure-activity relationship"**

(73) Proprietor: **ELI LILLY AND COMPANY**
**307, East McCarty Street**
**Indianapolis Indiana 46285 (US)**

(72) Inventor: **Debono, Manuel**
**5257 Hinesley Avenue**
**Indianapolis Indiana 46208 (US)**
Inventor: **Kirst, Herbert Andrew**
**1819 Madison Village Drive, Apt. 8-6**
**Indianapolis Indiana 46227 (US)**

(74) Representative: **Crowther, Terence Roger et al**
**Erl Wood Manor**
**Windlesham Surrey GU20 6PH (GB)**

EP 0 103 465 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to macrolide antibiotics, and more specifically to a novel group of C—20 modified derivatives of tylosin, desmycosin, macrocin, lactenocin, 2‴-O-demethylmacrocin (DOMM), 2″-O-demethylacetenocin (DOML), dihydrotylosin (relomycin), dihydrodesmycosin, dihydromacrocin, dihydrolactenocin, dihydro-DOMM, dihydro-DOML, and 4′-deoxydesmycosin, which are useful as antibiotics and as intermediates to antibiotics.

Improved antibiotics are continually in demand. In addition to antibiotics which are useful for treating human diseases, improved antibiotics are also needed in the veterinary field. Increased potency, expanded spectrum of bacterial inhibition, increased *in vivo* efficacy, and improved pharmaceutical properties (such as greater oral absorption, higher blood or tissue concentrations, longer body half life, and more advantageous rate or route of excretion and rate or pattern of metabolism) are some of the goals for improved antibiotics.

Tylosin is a well-known therapeutic agent in the veterinary field. (See, for example, *Tetrahedron Letters 1970*, 2339 and U.S. Patent No. 3,178,341). Tylosin and tylosin-like macrolides have been modified in an attempt to obtain derivatives having improved properties. A large number of derivatives have been made, but improvement in activity has not previously been obtained to the desired degree.

Certain 20-amino tylosin derivatives are disclosed in EP—A—87921, but none wherein the amino group is a heterocycle containing a single nitrogen atom.

We have now discovered that reductive amination of the C—20 aldehyde group of tylosin and the aforementioned tylosin-like macrolides using certain cyclic amines as aminating agents results in derivatives with significantly increased activity.

More specifically, the invention provides macrolide derivatives of the formula (I)

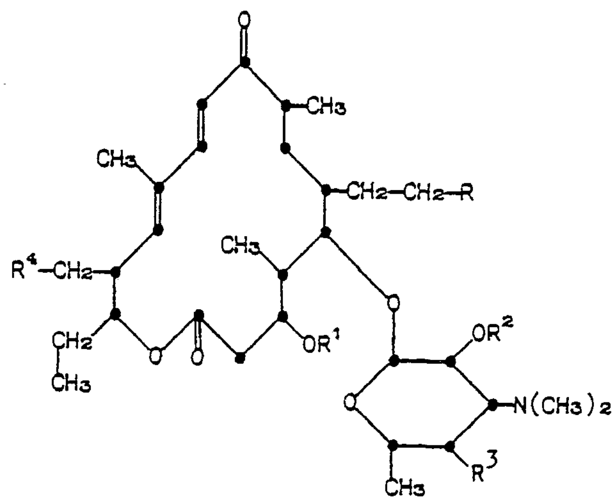

wherein

R is a monocyclic saturated or unsaturated ring containing one nitrogen atom as a sole heteroatom, the ring being bonded through the nitrogen atom and including from 5 to 16 ring atoms, or a bicyclic or tricyclic saturated or unsaturated ring containing one nitrogen atom as the sole heteroatom, the ring being bonded through the nitrogen atom and including from 8 to 20 ring atoms, the monocyclic, bicyclic, or tricyclic ring being optionally substituted at one or more of the carbon atoms by

- $C_1$—$C_4$ alkyl,
- hydroxyl,
- di-($C_1$—$C_4$ alkyl)amino,
- $-N(CH_2)_m$, where m = 4—7,
- $-\overset{\overset{O}{\|}}{C}N(C_1-C_4\ alkyl)_2$,
- $-\overset{\overset{O}{\|}}{C}N(CH_2)_m$, where m = 4—7,
- $C_1$—$C_4$ alkoxycarbonyl,
- phenyl,
- phenyl substituted by 1, 2, or 3 groups selected from nitro, halo, $C_1$—$C_4$ alkyl, $C_1$—$C_4$ alkoxy, hydroxy, amino, and mono or di-($C_1$—$C_4$ alkyl)amino,
- benzyl,

$C_2$—$C_4$ alkenyl,
$C_2$—$C_4$ alkynyl,
$C_1$—$C_4$ alkoxy,
$C_1$—$C_4$ alkanoyloxy,
halo,
halo-($C_1$—$C_4$ alkyl)-,
cyano,
ethylenedioxy;

$R^1$ is hydrogen or a hydroxyl protecting group;

$R^2$ is hydrogen, optionally substituted $C_1$—$C_5$-alkanoyl, optionally substituted phenylacetyl, optionally substituted phenylpropionyl, or optionally substituted benzoyl;

$R^3$ is hydrogen, iodo, hydroxy, optionally substituted $C_1$—$C_5$-alkanoyloxy, optionally substituted benzoyloxy, optionally substituted phenylacetoxy, optionally substituted phenoxyacetoxy; or

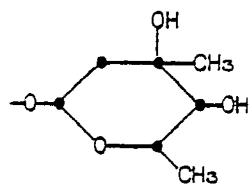

(mycarosyloxy)

$R^4$ is

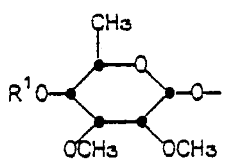
;
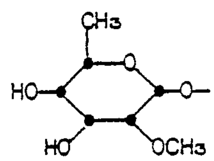
; or
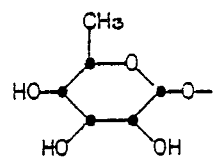

The groups of formula $R^4$ having the same conformation as the mycinosyloxy group of tylosin.

Wherein $R^1$ is as defined above, or an acid addition salt thereof, provided that if $R^3$ is hydrogen or iodo then $R^4$ is

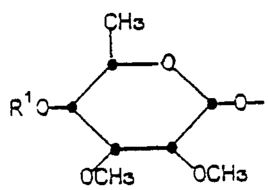

When R is an unsaturated ring, representative groups are 1,2,5,6-tetrahydropyridin-1-yl; 1,2,3,4-tetrahydroquinolin-1-yl; 1,2,3,4-tetrahydroisoquinolin-2-yl; indol-1-yl; isoindol-2-yl; indolin-1-yl; isoindolin-2-yl; 2,3,4,5-tetrahydro-1H-1-benzazepin-1-yl; 2,3,4,5-tetrahydro-1H-2-benzazepin-2-yl; 2,3,4,5-tetrahydro-1H-3-benzazepin-3-yl; pyrrol-1-yl; 1H-azepin-1-yl; carbazol-9-yl; acridin-10-yl; and acridin-9-one-10-yl.

When R is a saturated bicyclic or tricyclic ring, representative groups include decahydroquinolin-1-yl; decahydroisoquinolin-2-yl; decahydrocyclohepta[b]-pyrrol-1-yl; decahydrocyclohepta[c]pyrrol-2-yl; decahydrocyclopent[c]azepin-2-yl; deachydrocyclopent[d]azepin-3-yl; an azabicycloheptanyl group such as 3-azabicyclo[3.2.0]heptan-3-yl; an azabicyclooctanyl group such as 6-azabicyclo[3.2.1]octan-6-yl; an azabicyclononanyl group such as 3-azabicyclo[3.2.2]nonan-3-yl; an azabicyclodecanyl group such as 4-azabicyclo[5.3.0]decan-4-yl; an azatricyclotetradecanyl group such as 2-azatricyclo[6.2.2.$2^{3,6}$]tetradecan-2-yl; 1-azaspiro[4.5]decan-1-yl; and dodecahydrocarbazol-9-yl.

When R is a substituted ring representative R groups include 1,3,3 - trimethyl - 6 - azabicyclo[3.2.1]octan - 6 - yl; 4 - piperidinopiperidin - 1 - yl; 3,3,5 - trimethylhexahydroazepin - 1 - yl; 4 - hydroxypiperidin - 1 - yl; 3 - (N,N - diethylcarbamoyl)piperidin - 1 - yl; and the like.

In moieties containing a "$C_1$—$C_4$-alkyl group", the alkyl group can be straight, branched, or cyclic.

The term "$C_1$—$C_5$-alkanoyl" as used herein refers to an acyl moiety derived from a carboxylic acid containing from one to five carbon atoms. When optionally substituted, the alkyl group can bear one to three halo substituents. Halo substituents are selected from the group consisting of Cl, Br and F. Acetyl, chloroacetyl, trichloroacetyl, trifluoroacetyl, propionyl, *n*-butyryl, isobutyryl, *n*-valeryl, and isovaleryl are examples of such groups. The term "$C_1$—$C_5$-alkanoyloxy; refers to the corresponding acyloxy moiety.

The terms "optionally substituted benzoyl, phenylacetyl or phenylpropionyl" and "optionally substituted benzoyloxy, phenylacetoxy or phenoxyacetoxy" mean that the phenyl portion of the moiety is optionally substituted by from one to five halo or methyl groups or by from one to two methoxyl, nitro or hydroxyl groups.

The term "hydroxyl-protecting group" refers to a substituent which is not removed under the reaction conditions but which can be readily removed after the reaction has been completed to liberate the original hydroxyl group. Hydroxyl-protecting groups are well known in the art (see, for example, T. W. Greene, "Protective Groups in Organic Synthesis", Wiley-Interscience, 1981, pp. 10—86). One especially suitable hydroxy-protecting group is the tetrahydropyranyl group.

Preparing Macrolides of Formula (I)

Macrolides of formula (I) are prepared from corresponding macrolides of formula (II)

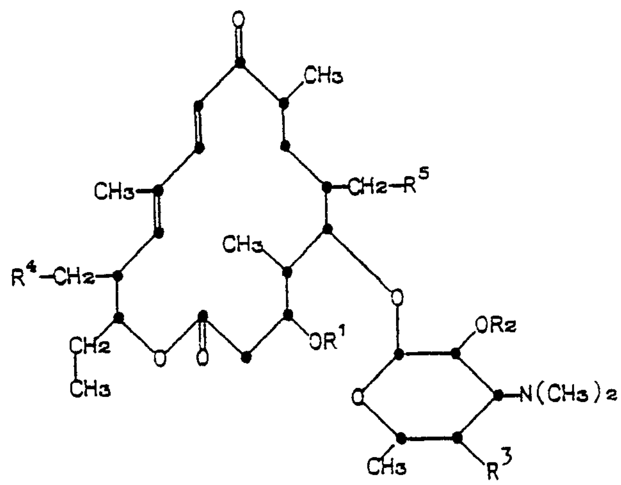

(II)

where $R^1$, $R^2$, $R^3$, and $R^4$ are as previously defined in formula (I) and $R^5$ is —CHO or —$CH_2L$. L represents a leaving group capable of undergoing displacement by an amine of formula HR where R is as defined in formula (I).

Thus, in accordance with one aspect of the invention, macrolides of formula (I) are prepared by reductive amination of aldehydes of formula (II) wherein $R^5$ is —CHO. Aldehyde starting materials include the following known macrolides of formula (II):

| | $R^1$ | $R^2$ | $R^3$ | $R^4$ |
|---|---|---|---|---|
| tylosin | H | H | mycarosyloxy | mycinosyloxy |
| desmycosin | H | H | OH | mycinosyloxy |
| 4'-deoxydesmycosin | H | H | H | mycinosyloxy |
| macrocin | H | H | mycarosyloxy | 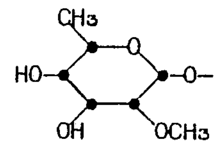 |
| lactenocin | H | H | OH | 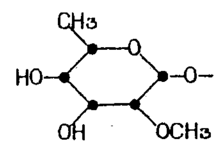 |
| 2'''-O-demethylmacrocin (DOMM) | H | H | mycarosyloxy | 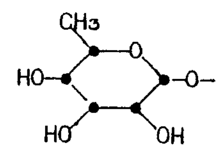 |
| 2'''-O-demethylactenocin (DOML) | H | H | OH | 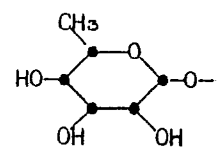 |

Tylosin and its preparation by fermentation of *Streptomyces fradiae* NRRL 2702 or 2703 are described in U.S. Patent No. 3,178,341. Desmycosin and preparation of desmycosin by mild acid hydrolysis of tylosin are also described in U.S. Patent No. 3,178,341.

4'-Deoxydesmycosin and a method of preparing it from desmycosin are described in A. Tanaka et al., *J. Antibiotics 34,* 1381—84 (1981).

Macrocin and its preparation by fermentation of *Streptomyces fradiae* NRRL 2702 or 2703 are described in U.S. Patent No. 3,326,759. Lactenocin and preparation of lactenocin by mild acid hydrolysis of macrocin are also described in U.S. Patent No. 3,326,759.

DOMM and its preparation by fermentation of *Streptomyces fradiae* ATCC 31669 are described in EPO published specification 45157. DOML and preparation of DOML by mild acid hydrolysis of DOMM are also described in EPO published specification 45157.

Aldehyde starting materials of formula (II) which are 2'- or 4'- monoesters, or 2',4'-diesters of the above described known macrolides, i.e., macrolides of formula (II) wherein $R^2$ is other than hydrogen or $R^3$ is other than hydroxyl, or both, are prepared by known acylation procedures. Typical acylating agents include activated carboxylic acid derivatives such as anhydrides, acid halides (usually in combination with a base or other acid scavenger) and active esters. Suitable organic solvents for this reaction include pyridine and triethylamine. Acylation can also be achieved using a mixture of an organic acid and a dehydrating agent such as N,N'-dicyclohexylcarbodiimide. Once formed, the acyl derivative can be separated and purified by known techniques.

Aldehyde starting materials of Formula (II) which are 2'-monoester derivatives can be prepared by selective esterification of compounds of formula (II) wherein $R^2$ is hydrogen and $R^3$ is hydroxyl using the techniques described in U.S. Patents 4,321,361 and 4,321,362. 3'-Hydroxyl groups are more facile to esterification than 4'-hydroxyl groups. Accordingly, the 2'-monoester derivative can be selectively prepared by, for example, treating the macrolide starting material with a stoichiometric quantity (or a slight excess) of an acylating agent, such as an acyl anhydride, at about room temperature for from about 1 to about 24 hours until esterification is substantially complete. The 2'-monoester can be isolated from the reaction mixture by standard procedures such as extraction, chromotography and crystallization.

Aldehyde starting materials which are 2',4'-diester derivatives are prepared from macrolides of formula (II) wherein $R^2$ is hydrogen and $R^3$ is hydroxyl using the procedure described in published

European Patent specification 82,003. Thus, symmetrical 2',4'-diester derivatives are prepared by treating the known macrolide of formula (II) wherein $R^2$ is hydrogen and $R^3$ is hydroxyl with a stoichiometric quantity (or a slight excess) of an acylating agent, such as an acyl anhydride, in a neutral solvent such as acetone, at about room temperature for from 1 to about 24 hours until esterification of the 2' and 4' hydroxyl groups is complete. Unsymmetrical 2',4'-diester derivatives, i.e., compounds of formula (II) wherein $OR^2$ and $R^3$ are different, can be prepared by acylation of appropriate 2'-monoesters.

The aldehyde starting materials of formula (II) are converted to the amines of formula (I) by reduction in the presence of an amine of formula HR, where R is as defined in formula (I). The preferred reducing agent is a cyanoborohydride of formula $MBH_3CN$ where M is a group 1A metal or ammonium. Sodium cyanoborohydride is the reducing agent of choice. The reaction is preferably conducted using an excess of the amine of formula HR, typically from 2 to 3 equivalents. The solvent for the reaction will normally be an inert polar solvent such as a $C_1$—$C_4$ alkanol, preferably methanol. The reaction temperature may be from 0 to 60°C., preferably 20 to 40°C. Neutral conditions (pH 6 to 8) are preferred. Dehydrating agents such as 4A molecular sieve or anhydrous sodium or magnesium sulfate can advantageously be used in the reaction.

Amines of formula (I) can also be prepared by reacting an amine of the formula HR where R is as defined in formula (I) with a macrolide of formula (II) where $R^5$ is —$CH_2L$ and L is a leaving group capable of undergoing displacement by the amine. Suitable leaving groups include, for example, trifluoromethanesulfonyl (triflate), and iodo.

Starting materials of formula (II) wherein $R^5$ is —$CH_2L$ are conveniently prepared from the following known macrolides of formula (II) wherein $R^5$ is —$CH_2OH$:

| | $R^1$ | $R^2$ | $R^3$ | $R^4$ |
|---|---|---|---|---|
| dihydrotylosin (relomycin) | H | H | mycarosyloxy | mycinosyloxy |
| dihydrodesmycosin | H | H | OH | mycinosyloxy |
| dihydromacrocin | H | H | mycarosyloxy | 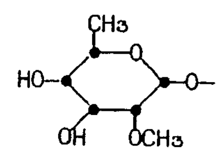 |
| dihydrolactenocin | H | H | OH | 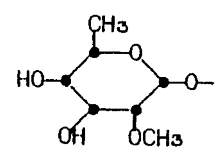 |
| dihydro-DOMM | H | H | mycarosyloxy | 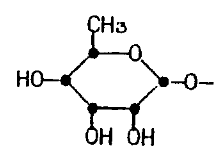 |
| dihydro-DOML | H | H | OH | 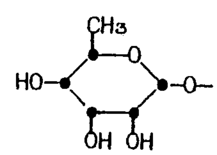 |

These macrolides are prepared by reducing the aldehyde group of tylosin, desmoycosin, macrocin, lactenocin, DOMM, and DOML, respectively.

The C—20 hydroxyl group of the foregoing macrolides are then converted to the desired leaving group L by methods known per se. For example, the C—20 hydroxyl group can be converted to the triflate group by reacting the macrolide with an activated derivative of trifluoromethane sulfonic acid such as trifluoromethane sulfonic anhydride or trifluoromethane sulfonylchloride in the presence of a base in a non-reactive organic solvent. If desired, the triflate group may be converted to the iodo group, for example by reacting the unisolated sulfonic ester intermediate with a source of iodide ion such as tetra n-butylammonium iodide, or sodium iodide. In case of dihydrodesmycosin, dihydrolactenocin, and dihydro-DOML, the 20-iodo derivative can be formed directly by adding iodine dissolved in a suitable solvent, such as dimethylformamide, to a solution of the 20-dihydro macrolide derivative and triphenylphosphine under nitrogen.

The leaving group at C—20 can then be displaced by reaction with the amine HR in a suitable nonreactive organic solvent such as acetonitrile, to yield compounds of formula (I).

When a product of formula (I) is one in which $R^3$ is mycarosyloxy, the corresponding macrolide of formula (I) wherein $R^3$ is hydroxyl can be prepared by acid hydrolysis of the initial product. More specifically, the mycarose sugar can be hydrolytically cleaved at pH of less than 4, preferably in the range from 0.5 to 2.0, at a temperature in the range from 0 to 60°C, conveniently at room temperature. The hydrolysis can be effected using a strong aqueous mineral acid as hydrochloric or sulfuric acid or a strong organic acid such as *p*-toluenesulfonic acid.

As previously mentioned, a method of preparing 4'-deoxydesmycosin is described in *J. of Antibiotics 34,* 1381—84 (1981). The process involves (1) treatment of desmycosin with acidic ethanol in accordance with a procedure obtained in *Antibiot. & Chemoth. 11,* 320—27 (1961), to obtain the corresponding diethylacetal; (2) acylation of the diethylacetal with acetic anhydride in acetonitrile in the absence of external base, in accordance with a procedure described in *J. Org. Chem. 44,* 2050—52 (1979), to obtain the 2',4'-di-O-acetyl derivative; (3) reacting the 2',4'-di-O-acetyl derivative with 2,3-dihydrofuran in dichloromethane in the presence of pyridinium p-toluenesulfonate in the manner described in *J. Org. Chem. 42,* 3772—74 (1974) to obtain the 3,4''-bis(O-tetrahydrofuranyl)derivative; (4) removal of the 2' and 4'-O-acetyl groups by dissolving the product of step (3) in methanol (50°C, overnight); (5) reacting the product of step (4) with 1.5 mole equivalent of benzenesulfonyl chloride in pyridine at −40°C for 4 hours, to provide the 4'-O-benzenesulfonyl derivative; (6) immediately reacting the 4'-O-benzenesulfonyl derivative with 1.5 equivalent of sodium iodide in methyl ethyl ketone at 180°C. for 15 minutes to obtain 4' iodo derivative; (7) reductively deiodinating the 4'-iodo derivative using tri(n-butyl)stannane in benzene in the presence of 2,2'-azobis-isobutyronitrile at 80°C for 2 hours; and (8) deblocking the diethylacetal and tetrahydrofuranyl groups by hydrolysis of the product of step (7) in .1M aqueous hydrochloric acid-acetonitrile (2.5:1 v/v) for 30 minutes at 25°C to obtain 4'-deoxydesmycosin.

The 4'-deoxydesmycosin thus prepared may then be modified at the C—20, and optionally at the 2' position as described above.

Alternatively, a C—20 modified derivative of 4'-deoxydesmycosin may be prepared by deoxygenating a C—20 modified derivative of desmycosin, for example by treating the C—20 modified derivative in accordance with steps 2 through 6 or 2 through 8 of the process of *J. Antibiotics 34,* 1381—84 (1981) as described above.

The procedures used to prepare 2' and 4' esters of the starting macrolides of formula (II) were described above. Macrolides of formula (I) can be acylated using identical procedures to obtain 2'- and 4'-monoesters and 2',4'-diesters of formula (I).

The C—20-modified derivatives of this invention form salts, particularly acid addition salts. These acid addition salts are also useful as antibiotics and are a part of this invention. In another aspect, such salts are useful as intermediates, for example, for separating and purifying the derivatives. In addition, the salts have an improved solubility in water.

Representative suitable salts include those salts formed by standard reactions with both organic and inorganic acids such as, for example, sulfuric, hydrochloric, phosphoric, acetic, succinic, citric, lactic, maleic, fumaric, palmitic, cholic, pamoic, mucic, D-glutamic, *d*-camphoric, glutaric, glycolic, phthalic, tartaric, formic, lauric, stearic, salicyclic, methanesulfonic, benzenesulfonic, sorbic, picric, benzoic, cinnamic, and like acids.

Accordingly, the present invention provides a process for preparing a macrolide of formula (I) or a pharmaceutically-acceptable salt thereof, which comprises

(a) reducing an aldehyde of formula (III)

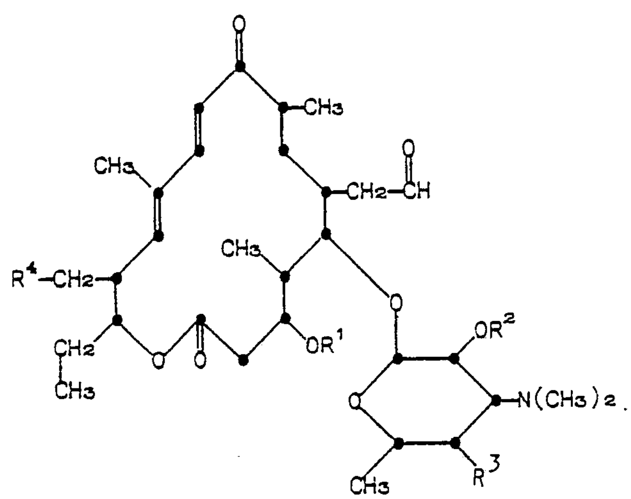

where $R^1$, $R^2$, $R^3$ and $R^4$ are as defined in formula (I) in the presence of an amine of formula HR where R is as defined in formula (I), or

(b) reacting an amine of formula HR, where R is as defined in formula (I) with a macrolide of formula (IV)

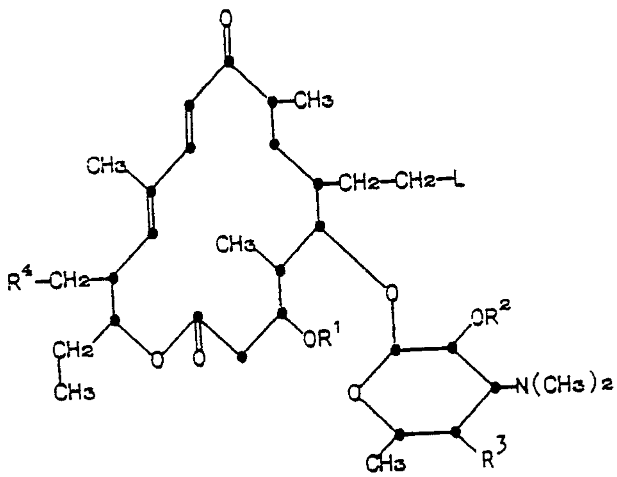

wherein L is a leaving group capable of being displaced by the amine HR, in a nonreactive organic solvent, or

(c) cleaving the mycarose sugar from a macrolide of formula (I) in which $R^3$ is mycarosyloxy by acid hydrolysis to provide a macrolide of formula (I) wherein $R^3$ is hydroxy, or

(d) converting a sulfonic ester of the formula (V)

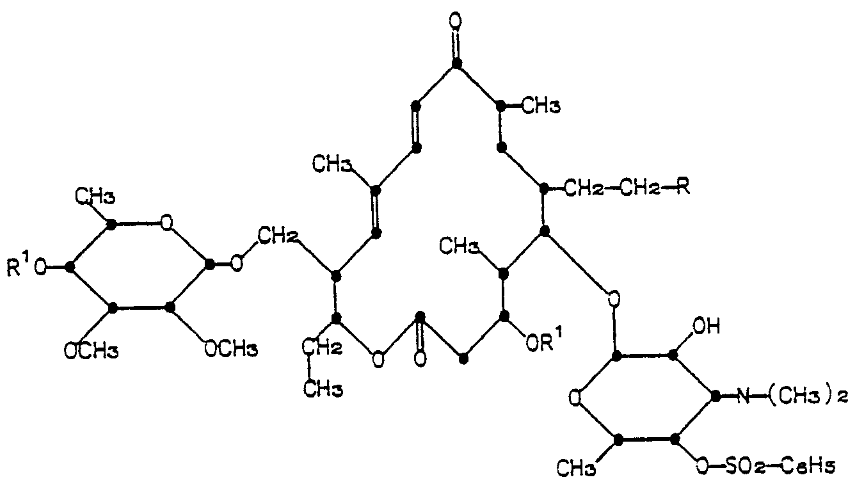

wherein R and $R^1$ are as defined in formula (I) to the corresponding 4'-iodide by reacting the sulfonic ester with a source of iodide ion in an inert organic solvent, and if $R^1$ is other than hydrogen, optionally hydrolyzing to provide the corresponding macrolide wherein $R^1$ is hydrogen or

(e) reductively deiodinating a macrolide of formula (VI)

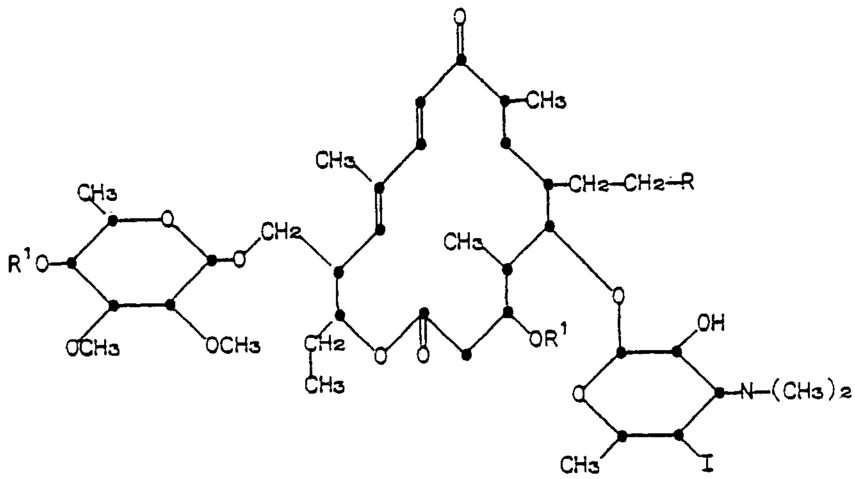

wherein R is as defined in formula (I) and each $R^1$ is a hydroxyl protecting group to produce a macrolide of formula (I) wherein $R^3$ is H, and optionally removing the hydroxyl protecting groups to provide a macrolide of formula (I) wherein $R^1$ and $R^3$ are hydrogen, and

(f) if necessary, esterifying or salifying, or both, a product of reaction (a), (b), (c), (d) or (e).

Pharmaceutically acceptable acid addition salts are an especially preferred group of salts of this invention.

Illustrative C—20-modified derivatives of this invention include the compounds listed in Tables I—VIII.

TABLE I
Illustrative C—20 Modified Derivatives of Tylosin[a]

| Compound No. | R |
|---|---|
| T1 | pyrrolidin-1-yl |
| T2 | piperidin-1-yl |
| T3 | 4-hydroxypiperidin-1-yl |
| T4 | 4-phenylpiperidin-1-yl |
| T5 | hexahydroazepin-1-yl |
| T6 | octahydroazocin-1-yl |
| T7 | octahydro-1H-azonin-1-yl |
| T8 | decahydroazecin-1-yl |
| T9 | azacycloundecan-1-yl |
| T10 | azacyclotridecan-1-yl |
| T11 | 1,2,3,4-tetrahydroquinolin-1-yl |
| T12 | 1,2,3,4-tetrahydroisoquinolin-2-yl |
| T13 | 3-azabicyclo[3.2.2]nonan-3-yl |

a $R^4$ = 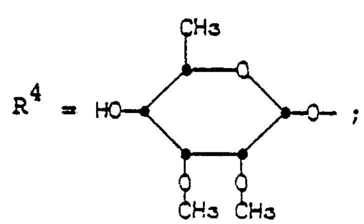 ; $R^2$ = H; and $R^3$ = mycarosyloxy

TABLE II
Illustrative C—20 Modified Derivatives of Desmycosin[a]

| Compound No. | R | $R^3$ |
| --- | --- | --- |
| D1 | pyrrolidin-1-yl | OH |
| D1a | pyrrolidin-1-yl | H |
| D2 | piperidin-1-yl | OH |
| D2a | piperidin-1-yl | H |
| D3 | 4-hydroxypiperidin-1-yl | OH |
| D3a | 4-hydroxypiperidin-1-yl | H |
| D4 | 4-phenylpiperidin-1-yl | OH |
| D4a | 4-phenylpiperidin-1-yl | H |
| D5 | hexahydroazepin-1-yl | OH |
| D5a | hexahydroazepin-1-yl | H |
| D6 | octahydroazocin-1-yl | OH |
| D6a | octahydroazocin-1-yl | H |
| D7 | octahydro-1-H-azonin-1-yl | OH |
| D7a | octahydro-1-H-azonin-1-yl | H |
| D8 | decahydroazecin-1-yl | OH |
| D8a | decahydroazecin-1-yl | H |
| D9 | azacycloundecan-1-yl | OH |
| D9a | azacycloundecan-1-yl | H |
| D10 | azacyclotridecan-1-yl | OH |
| D10a | azacyclotridecan-1-yl | H |
| D11 | 1,2,3,4-tetrahydroquinolin-1-yl | OH |
| D11a | 1,2,3,4-tetrahydroquinolin-1-yl | H |
| D12 | 1,2,3,4-tetrahydroisoquinolin-2-yl | OH |
| D12a | 1,2,3,4-tetrahydroisoquinolin-2-yl | H |
| D13 | 4-piperidinopiperidin-1-yl | OH |
| D13a | 4-piperidinopiperidin-1-yl | H |

a $R^4$ = 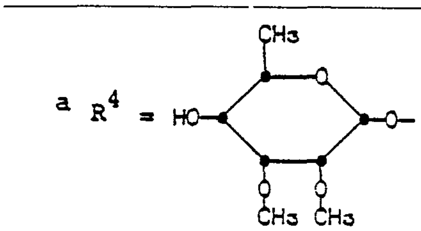 ; $R^2$ = H; and $R^3$ = OH

TABLE II continued

| Compound No. | R | $R^3$ |
|---|---|---|
| D14 | 3-azabicyclo[3.2.2]nonan-3-yl | OH |
| D14a | 3-azabicyclo[3.2.2]nonan-3-yl | H |
| D15 | 3-(N,N-diethylcarbamoyl)piperidin-1-yl | OH |
| D15a | 3-(N,N-diethylcarbamoyl)piperidin-1-yl | H |
| D16 | 4-(N,N-dimethylamino)hexahydroazepin-1-yl | OH |
| D16a | 4-(N,N-dimethylamino)hexahydroazepin-1-yl | H |
| D17 | 2-azabicyclo[2.2.2]octan-2-yl | OH |
| D17a | 2-azabicyclo[2.2.2]octan-2-yl | H |
| D18 | decahydrocyclopent[d]azepin-3-yl | OH |
| D18a | decahydrocyclopent[d]azepin-3-yl | H |
| D19 | 1-azaspiro[4.5]decan-1-yl | OH |
| D19a | 1-azaspiro[4.5]decan-1-yl | H |
| D20 | decahydroquinolin-1-yl | OH |
| D20a | decahydroquinolin-1-yl | H |
| D21 | 1,3,3-trimethyl-6-azabicyclo[3.2.1]octan-6-yl | OH |
| D21a | 1,3,3-trimethyl-6-azabicyclo[3.2.1]octan-6-yl | H |
| D22 | 1,2,3,6-tetrahydropyridin-1-yl | OH |
| D22a | 1,2,3,6-tetrahydropyridin-1-yl | H |
| D23 | 3,3,5-trimethylhexahydroazepin-1-yl (isomer 1) | OH |
| D23a | 3,3,5-trimethylhexahydroazepin-1-yl (isomer 1) | H |
| D24 | 3,3,5-trimethylhexahydroazepin-1-yl (isomer 2) | OH |
| D24a | 3,3,5-trimethylhexahydroazepin-1-yl (isomer 2) | H |
| D25 | dodecahydrocarbazol-9-yl | OH |
| D25a | dodecahydrocarbazol-9-yl | H |
| D26 | 4-phenyl-1,2,3,6-tetrahydropyridin-1-yl | OH |
| D26a | 4-phenyl-1,2,3,6-tetrahydropyridin-1-yl | H |
| D27 | 4-benzyl-piperidin-1-yl | OH |
| D27a | 4-benzyl-piperidin-1-yl | H |
| D28 | 4-(ethylenedioxy)piperidin-1-yl | OH |
| D28a | 4-(ethylenedioxy)piperidin-1-yl | H |
| D29 | decahydroisoquinolin-2-yl | OH |

TABLE II continued

| Compound No. | R | $R^3$ |
|---|---|---|
| D29a | decahydroisoquinolin-2-yl | H |
| D30 | 7-azabicyclo[2.2.1]heptan-7-yl | OH |
| D30a | 7-azabicyclo[2.2.1]heptan-7-yl | H |
| D31 | Pyrrol-1-yl | OH |
| D31a | Pyrrol-1-yl | H |
| D32 | Carbazol-9-yl | OH |
| D32a | Carbazol-9-yl | H |

TABLE III
Illustrative C—20 Modified Derivatives of Macrocin[a]

| Compound No. | R |
|---|---|
| M1 | pyrrolidin-1-yl |
| M2 | 4-phenylpiperidin-1-yl |
| M3 | hexahydroazepin-1-yl |
| M4 | octahydroazocin-1-yl |
| M5 | octahydro-1H-azonin-1-yl |
| M6 | azacyclotridecan-1-yl |
| M7 | 1,2,3,4-tetrahydroisoquinolin-2-yl |
| M8 | 3-azabicyclo[3.2.2]nonan-3-yl |

a $R^4$ = 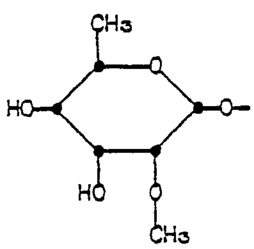 ; $R^2$ = H; and $R^3$ = mycarosyloxy

TABLE IV
Illustrative C—20 Modified Derivatives of Lactenocin[a]

| Compound No. | R |
|---|---|
| L1 | pyrrolidin-1-yl |
| L2 | piperidin-1-yl |
| L3 | 4-phenylpiperidin-1-yl |
| L4 | hexahydroazepin-1-yl |
| L5 | octahydroazocin-1-yl |
| L6 | octahydro-1H-azonin-1-yl |
| L7 | azacyclotridecan-1-yl |
| L8 | 1,2,3,4-tetrahydroisoquinolin-2-yl |
| L9 | 3-azabicyclo[3.2.1]nonan-3-yl |
| L10 | 1,3,3-trimethyl-6-azabicyclo[3.2.1]octan-6-yl |

[a] $R^4$ = 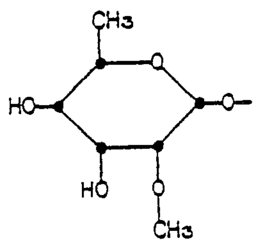 ; $R^2$ = H; $R^3$ = OH

TABLE V
Illustrative C—20-Modified Derivatives of DOMM[a]

| Compound No | R |
|---|---|
| C1 | pyrrolidin-1-yl |
| C2 | 4-phenylpiperidin-1-yl |
| C3 | hexahydroazepin-1-yl |
| C4 | octahydroazocin-1-yl |
| C5 | azacyclotridecan-1-yl |

[a] $R^4$ = 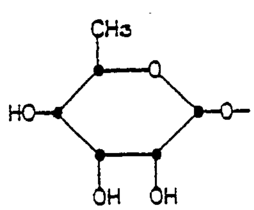 ; $R^2$ = H; and $R^3$ = macarosyloxy

TABLE VI
Illustrative C—20-Modified Derivatives of DOML[a]

| Compound No. | R |
|---|---|
| N1 | pyrrolidin-1-yl |
| N2 | 4-phenylpiperidin-1-yl |
| N3 | hexahydroazepin-1-yl |
| N4 | octahydroazocin-1-yl |
| N5 | octahydro-1H-azonin-1-yl |
| N6 | azacyclotridecan-1-yl |
| N7 | 1,2,3,4-tetrahydroquinolin-1-yl |

a $R^4$ = 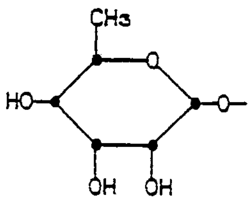 ; $R^2$ = H; $R^3$ = OH

TABLE VII
Illustrative C—20-Modified Ester Derivatives of Tylosin[a]

| Compound No. | R | $R^2$ (2')[b] |
|---|---|---|
| E1 | hexahydroazepin-1-yl | propionyl |
| E2 | octahydroazocin-1-yl | propionyl |
| E3 | piperidin-1-yl | acetyl |

a $R^4$ = 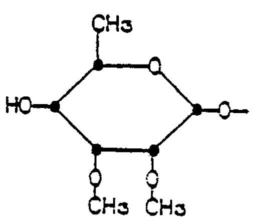 ; $R^3$ = mycarosyloxy

[b]position number of esterified hydroxyl group in parentheses

TABLE VIII
Illustrative C—20-Modified Ester Derivatives of Desmycosin[a]

| Compound No. | R | $R^2$ (2')[b] | $R^3$ (4')[b] |
|---|---|---|---|
| E4 | hexahydroazepin-1-yl | propionyl | H |
| E5 | hexahydroazepin-1-yl | propionyl | propionyl |
| E6 | octahydroazocin-1-yl | acetyl | H |
| E7 | octahydroazocin-1-yl | acetyl | acetyl |
| E8 | piperidin-1-yl | propionyl | H |

[a] $R^4$ = 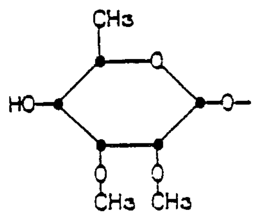 ; [b]position number of esterified hydroxyl group in parentheses

The derivatives of this invention inhibit the growth of pathogenic bacteria, especially gram-positive bacteria, *Mycoplasma* species and gram-negative bacteria such as *Pasteurella* species. The derivatives are particularly useful against *Pasteurella* species such as *P. multocida* and *P. hemolytica* and against *Mycoplasma* species such as *M. gallisepticum* and *M. hyopneumoniae* (the causative agent of mycoplasmal pneumonia in swine).

The minimal inhibitory concentrations (MIC's) at which illustrative compounds inhibit certain bacteria are given in Tables IX and X. The MIC's in Table IX were determined by standard agar-dilution assays. The MIC's in Table X were obtained using conventional broth-dilution microtiter tests.

TABLE IX
Antibiotic Activity of C—20 Modified Derivatives[a]

| Test Organism | Test Compound[b] | | | | | | | | |
|---|---|---|---|---|---|---|---|---|---|
| | D1 | D4 | D5 | D5a | D6 | D10 | D14 | M3 | L5 |
| *Staphylococcus aureus* X1.1 | 2 | 0.5 | 0.5 | 0.5 | 0.5 | 0.25 | 0.25 | 4 | 1 |
| *Staphylococcus aureus* V41[c] | 2 | 0.5 | 0.5 | 0.5 | 0.5 | 0.25 | 0.25 | 8 | 1 |
| *Staphylococcus aureus* X400[d] | 2 | 0.5 | 1 | 1 | 0.5 | 0.25 | 0.5 | 32 | 2 |
| *Staphylococcus aureus* S13E | 2 | 0.5 | 0.25 | 0.5 | 0.5 | 0.25 | 0.25 | 8 | 1 |
| *Staphylococcus epidermidis* EPI1 | 2 | 0.5 | 0.5 | 0.5 | 0.5 | 0.25 | 0.5 | 8 | 1 |
| *Staphylococcus epidermidis* EPI2 | 1 | 0.25 | 0.12 | 0.25 | 0.25 | 0.12 | 0.12 | 2 | 0.5 |
| *Streptococcus pyogenes* C203 | 1 | 0.25 | 0.5 | 0.25 | 0.5 | NT[h] | NT | 4 | 0.5 |
| *Streptococcus pneumoniae* Park I | 0.5 | 0.25 | 0.5 | 0.25 | 0.5 | 0.25 | 0.5 | 2 | 0.5 |
| *Streptococcus* Group D X66 | 32 | 1 | 8 | 2 | 4 | 4 | 4 | — | 16 |
| *Streptococcus* Group 9960 | 16 | 2 | 8 | 2 | 8 | 4 | 4 | — | 16 |
| *Haemophilus influenzae* C.L.[e] | 16 | 8 | 16 | 8 | 16 | 4 | 4 | 128 | 32 |
| *Haemophilus influenzae* 76[f] | 16 | 8 | 128 | 8 | 16 | 8 | 8 | 128 | 16 |
| *Escherichia coli* N10 | —[g] | 64 | 128 | 64 | 64 | 32 | 32 | — | — |
| *Escherichia coli* EC14 | — | 64 | 4 | 128 | 128 | 64 | 32 | — | — |
| *Escherichia coli* TEM | 128 | 32 | 8 | 8 | 16 | 8 | 16 | — | 16 |
| *Klebsiella* X26 | 16 | 32 | —[g] | 8 | 16 | 16 | 8 | — | 16 |

TABLE IX continued
Antibiotic Activity of C—20 Modified Derivatives[a]

| Test Organism | Test Compound[b] | | | | | | | | |
|---|---|---|---|---|---|---|---|---|---|
| | M4 | L4 | T6 | T13 | D2 | D3 | D7 | D11 | D12 |
| *Staphylococcus aureus* X1.1 | 4 | 2 | 4 | 4 | 0.5 | 8 | 0.5 | 0.5 | 0.5 |
| *Staphylococcus aureus* V41[c] | 4 | 2 | 4 | 4 | 0.5 | 8 | 0.5 | 0.5 | 0.5 |
| *Staphylococcus aureus* X400[d] | 8 | 2 | 8 | 8 | 1 | 16 | 0.5 | 0.5 | 0.5 |
| *Staphylococcus aureus* S13E | 4 | 2 | 4 | 4 | 0.5 | 8 | 0.5 | 0.5 | 0.5 |
| *Staphylococcus epidermidis* EPI1 | 4 | 2 | 4 | 4 | 1 | 8 | 0.5 | 0.5 | 0.5 |
| *Staphylococcus epidermidis* EPI2 | 2 | 1 | 2 | 2 | 0.5 | 4 | 0.25 | 0.25 | 0.25 |
| *Streptococcus pyogenes* C203 | 2 | 1 | 2 | 4 | 0.25 | 1 | 0.06 | 0.5 | 0.5 |
| *Streptococcus pneumoniae* Park I | 32 | 8 | 8 | 16 | 0.25 | 8 | 0.5 | 2 | 1 |
| *Streptococcus* Group D X66 | — | 32 | — | 128 | 16 | 128 | 4 | 8 | 4 |
| *Streptococcus* Group 9960 | — | 16 | — | — | 16 | 32 | 8 | 8 | 8 |
| *Haemophilus influenzae* C.L.[e] | 128 | 16 | 64 | 128 | 16 | 64 | 16 | 64 | 16 |
| *Haemophilus influenzae* 76[f] | 128 | 16 | — | 128 | 16 | 64 | 16 | 8 | 8 |
| *Escherichia coli* N10 | — | — | — | — | — | — | 64 | — | — |
| *Escherichia coli* EC14 | — | — | — | — | — | — | 64 | — | 128 |
| *Escherichia coli* TEM | — | — | — | — | 16 | — | 16 | — | 64 |
| *Klebsiella* X26 | — | 16 | — | — | 16 | 64 | — | — | 32 |

TABLE IX continued
Antibiotic Activity of C—20 Modified Derivatives[a]

| Test Organism | Test Compound[b] | | | | | | | | |
|---|---|---|---|---|---|---|---|---|---|
| | D13 | D15 | D19 | D20 | D21 | D22 | D23 | D24 | D25 |
| *Staphylococcus aureus* X1.1 | 0.5 | 2 | 1 | 0.5 | 1 | 1 | 0.5 | 0.5 | 0.5 |
| *Staphylococcus aureus* V41[c] | 1 | 2 | 1 | 0.5 | 1 | 0.5 | 0.5 | 0.5 | 0.5 |
| *Staphylococcus aureus* X400[d] | 1 | 4 | 1 | 0.5 | 1 | 1 | 1 | 1 | 1 |
| *Staphylococcus aureus* S13E | 1 | 2 | 1 | 0.5 | 1 | 0.5 | 0.5 | 0.5 | 0.5 |
| *Staphylococcus epidermidis* EPI1 | 1 | 2 | 1 | 0.5 | 1 | 0.5 | 0.5 | 0.5 | 0.5 |
| *Staphylococcus epidermidis* EPI2 | 0.25 | 1 | 0.25 | 0.25 | 0.5 | 0.25 | 0.5 | 0.25 | 0.25 |
| *Streptococcus pyogenes* C203 | 2 | 0.5 | 0.5 | 0.25 | 0.5 | 0.25 | 0.5 | 0.25 | 0.25 |
| *Streptococcus pneumoniae* Park I | 2 | 1 | 0.25 | 0.12 | 0.5 | 1 | 0.5 | 1 | 0.5 |
| *Streptococcus* Group D X66 | 2 | 32 | 16 | 8 | 4 | 8 | 4 | 4 | 4 |
| *Streptococcus* Group 9960 | 2 | 32 | 16 | 8 | 8 | 8 | 8 | 8 | 4 |
| *Haemophilus influenzae* C.L.[e] | 16 | 32 | 16 | 16 | 32 | 16 | 16 | 32 | 16 |
| *Haemophilus influenzae* 76[f] | 8 | 32 | 16 | 16 | 16 | 8 | 8 | 8 | 8 |
| *Escherichia coli* N10 | — | — | 128 | 128 | 64 | — | 64 | 64 | 128 |
| *Escherichia coli* EC14 | — | — | 128 | 128 | 64 | — | 64 | 64 | 128 |
| *Escherichia coli* TEM | — | 64 | 32 | 32 | 32 | 64 | 32 | 32 | 32 |
| *Klebsiella* X26 | 16 | 32 | 32 | 16 | 64 | 64 | 128 | 128 | 128 |

TABLE IX continued
Antibiotic Activity of C—20 Modified Derivatives[a]

| Test Organism | Test Compound[b] | | |
|---|---|---|---|
| | D26 | D27 | D28 |
| *Staphylococcus aureus* X1.1 | 0.5 | 1 | 2 |
| *Staphylococcus aureus* V41[c] | 0.5 | 1 | 2 |
| *Staphylococcus aureus* X400[d] | 0.5 | 1 | 4 |
| *Staphylococcus aureus* S13E | 0.5 | 1 | 2 |
| *Staphylococcus epidermidis* EPI1 | 0.5 | 1 | 2 |
| *Staphylococcus epidermidis* EPI2 | 0.25 | 0.25 | 1 |
| *Streptococcus pyogenes* C203 | 0.25 | 0.25 | 0.5 |
| *Streptococcus pneumoniae* Park I | 0.25 | 0.5 | 2 |
| *Streptococcus* Group D X66 | 1 | 2 | 8 |
| *Streptococcus* Group 9960 | 2 | 4 | 8 |
| *Haemophilus influenzae* C.L.[e] | 32 | 32 | 32 |
| *Haemophilus influenzae* 76[f] | 8 | 16 | 32 |
| *Escherichia coli* N10 | 128 | 128 | — |
| *Escherichia coli* EC14 | 128 | 128 | — |
| *Escherichia coli* TEM | 32 | 32 | 64 |
| *Klebsiella* X26 | 64 | 64 | 64 |

Footnotes to Table IX

[a] MIC in mcg/ml
[b] Compound numbers from Tables I—IV
[c] Penicillin-resistant strain
[d] Methicillin-resistant strain
[e] Ampicillin-sensitive strain
[f] Ampicillin-resistant strain
[g] Not active at 128 mcg/ml, the highest level tested
[h] Not tested

TABLE X
Antibiotic Activity of C—20 Modified Derivatives[a]

| Test Organism | Test Compound[b] | | | | | | | | | |
|---|---|---|---|---|---|---|---|---|---|---|
| | D1 | D2 | D3 | D4 | D5 | D6 | D7 | D10 | D11 | D12 |
| *Staphylococcus aureus* | 0.78 | 0.78 | 12.5 | 0.39 | 1.56 | 0.78 | 1.56 | 0.39 | 1.56 | 1.56 |
| *Streptococcus* sp. 80 | 6.25 | 6.25 | 12.5 | 0.09 | 3.12 | 0.78 | 3.12 | 0.78 | 3.12 | 3.12 |
| *Pasteurella multocida* 17E[c] | 6.25 | 25 | 50 | 6.25 | 3.12 | 3.12 | 6.25 | 1.56 | 12.5 | 6.25 |
| *Pasteurella multocida* 60A[d] | 6.25 | 6.25 | 50 | 6.25 | 3.12 | 6.25 | 6.25 | 3.12 | 50 | 12.5 |
| *Pasteurella hemolytica* 22C | 6.25 | 3.12 | 50 | 6.25 | 1.56 | 1.56 | 3.12 | 1.56 | 25 | 3.12 |
| *Mycoplasma gallisepticum* | 12.5 | 3.12 | 25 | 0.39 | 1.56 | 1.56 | 0.39 | 0.097 | ≤0.048 | ≤0.048 |
| *Mycoplasma synoviae* | 0.39 | 0.78 | 6.25 | <0.05 | 0.39 | 0.78 | 0.39 | ≤0.048 | 0.78 | 0.39 |
| *Mycoplasma hyorhinis* | 50 | >50 | 50 | 50 | 50 | 50 | 25 | 12.5 | 6.25 | 12.5 |
| *Mycoplasma hyopneumoniae* | 12.5 | 12.5 | 6.25 | 0.78 | 1.56 | 1.56 | 1.56 | 0.78 | 1.56 | 3.12 |

TABLE X cont'd.
Antibiotic Activity of C—20 Modified Derivatives[a]

| Test Organism | Test Compound[b] | | | | | | | | | | |
|---|---|---|---|---|---|---|---|---|---|---|---|
| | D13 | D14 | D15 | D19 | D20 | D21 | D22 | D23 | D24 | D25 | L5 |
| *Staphylococcus aureus* | 3.12 | 0.78 | 6.25 | 0.78 | 0.78 | 0.78 | 1.56 | 3.12 | 3.12 | 3.12 | 1.56 |
| *Streptococcus* sp. 80 | 6.25 | 1.56 | 12.5 | 3.12 | 3.12 | 1.56 | 3.12 | 1.56 | 3.12 | 3.12 | 0.78 |
| *Pasteurella multocida* 17E[c] | 12.5 | 3.12 | 12.5 | 3.12 | 3.12 | 3.12 | 12.5 | 6.25 | 12.5 | 12.5 | 6.25 |
| *Pasteurella multocida* 60A[d] | 12.5 | 3.12 | 12.5 | 3.12 | 3.12 | 3.12 | 12.5 | 12.5 | 6.25 | 12.5 | 12.5 |
| *Pasteurella hemolytica* 22C | 12.5 | 1.56 | 25 | 3.12 | 3.12 | 3.12 | 3.12 | 3.12 | 6.25 | 3.12 | 12.5 |
| *Mycoplasma gallisepticum* | 6.25 | 1.56 | 3.12 | 1.56 | 0.78 | 1.56 | 0.78 | 0.097 | 0.39 | 0.39 | 6.25 |
| *Mycoplasma synoviae* | 1.56 | 0.39 | 1.56 | 0.39 | 0.195 | 0.39 | 0.78 | 0.097 | 0.097 | 0.097 | 3.12 |
| *Mycoplasma hyorhinis* | 50 | 50 | >50 | >50 | 50 | — | 25 | 6.25 | 25 | 12.5 | 50 |
| *Mycoplasma hyopneumoniae* | 3.12 | 0.78 | 3.12 | 3.12 | 0.78 | 0.195 | 1.56 | 0.78 | 0.78 | 0.39 | NT[e] |

[a] MIC in mcg/ml
[b] Compound numbers from Tables II and IV
[c] Bovine isolate
[d] Avian isolate
[e] Not tested

TABLE X cont'd.
Antibiotic Activity of C—20 Modified Derivatives[a]

| Test Organism | Test Compound[b] | | | | | | | | |
|---|---|---|---|---|---|---|---|---|---|
| | D26 | D27 | D28 | D5a | L4 | T6 | T13 | M3 | M4 |
| *Staphylococcus aureus* | 3.12 | 3.12 | 3.12 | 0.39 | 6.25 | 12.5 | 6.25 | 12.5 | 12.5 |
| *Streptococcus* sp. 80 | 0.78 | 0.78 | 3.12 | 0.78 | 6.25 | 50 | 50 | 50 | 12.5 |
| *Pasteurella multocida* 17E[c] | 6.25 | 6.25 | 6.25 | 6.25 | 12.5 | >50 | >50 | >50 | >50 |
| *Pasteurella multocida* 60A[d] | 12.5 | 12.5 | 25 | 3.12 | 12.5 | >50 | >50 | >50 | >50 |
| *Pasteurella hemolytica* 22C | 12.5 | 12.5 | 25 | 3.12 | 25 | 50 | 50 | 50 | >50 |
| *Mycoplasma gallisepticum* | ≤0.048 | ≤0.048 | 0.097 | 0.78 | 12.5 | 3.12 | 1.56 | 6.25 | 3.12 |
| *Mycoplasma synoviae* | 0.78 | ≤0.048 | 0.195 | 0.195 | 1.56 | 0.78 | 1.56 | 25 | 6.25 |
| *Mycoplasma hyorhinis* | 25 | 25 | 25 | 3.12 | 50 | >50 | >50 | >50 | >50 |
| *Mycoplasma hyopneumoniae* | 0.78 | 0.39 | 1.56 | 0.195 | 1.56 | 12.5 | 25 | 12.5 | 6.25 |

[a] MIC in mcg/ml
[b] Compound numbers from Tables I—IV
[c] Bovine isolate
[d] Avian isolate
[e] Not tested

The C—20 modified derivatives of this invention have shown *in vivo* antimicrobial activity against experimentally-induced infections in laboratory animals. When two doses of test compound were administered to mice experimentally infected with *S. pyogenes* C203, the activity observed was measured as an $ED_{50}$ value [effective dose in mg/kg to protect 50% of the test animals: see Warren Wick, et al., *J. Bacteriol.* *81*, 233—235 (1961)]. $ED_{50}$ values observed for illustrative compounds are given in Table XI.

TABLE XI

$ED_{50}$ Values of C—20-Modified Derivatives vs. *Streptococcus pyogenes* C203 in Mice[a]

| Test Compound[b] | Subcutaneous | Oral |
|---|---|---|
| D1 | 1.2 | >50 |
| D2 | 0.9 | 50 |
| D4 | 6.0 | 19 |
| D5 | 1.3 | 50 |
| D5a | 1.5 | 34 |
| D6 | 0.7 | 14 |
| D7 | 1.6 | 12 |
| D10 | >10 | 68 |
| D11 | 7.5 | 19 |
| D12 | 2.0 | <6.3 |
| D14 | 1.0 | 50 |
| D19 | 2.9 | 46 |
| D20 | 1.7 | 34 |
| D21 | 1.0 | 10 |
| D22 | 0.8 | 40 |
| L5 | 1.8 | 100 |
| M3 | >10 | >100 |
| T6 | >10 | 44 |
| T13 | >10 | 30 |

[a] mg/kg × 2; doses given 1 and 4 hours post-infection
[b] Compound numbers from Tables I—IV.

Many of the C—20 modified derivatives of this invention have also shown *in vivo* antibacterial activity against infections induced by gram-negative bacteria. Tables XII and XIII summarize the results of tests in which illustrative compounds were evaluated against a *Pasteurella* infection in one-day-old chicks. The compounds were administered parenterally or orally after challenge of the chicks with *Pasteurella multocida* (0.1 ml of a $10^{-4}$ dilution of a twenty-hour tryptose broth culture of an avian *P. multocida* given subcutaneously). In these tests, unless indicated otherwise, all non-medicated infected chicks died within 24 hours of *Pasteurella* challenge. In the tests summarized in Table XII, the compounds were administered by subcutaneous injection at a dosage of 30 mg/kg, 1 and 4 hours post-challenge of the chicks with *P. multocida*. In the tests summarized in Table XIII the compounds were administered in medicated drinking water (at a level of 2 g/gal) available from 4 to 20 hours prior to challenge of the chicks with *P. multocida* and during the 3-day test period.

TABLE XII

Activity of C—20-Modified Derivatives Administered Subcutaneously to *Pasteurella multocida*-Infected Chicks[a]

| Test Compound[b] | Number of Deaths/Number Treated |
|---|---|
| D1 | 0/10 |
| D2 | 0/10 |
| D4 | 9/10 |
| D5 | 0/10 |
| D6 | 0/10 |
| D7 | 3/10 |
| D10 | 10/10 |
| D11 | 10/10 |
| D12 | 9/10 |
| D14 | 2/10 |
| D19 | 0/10 |
| D21 | 7/10 |
| D22 | 0/10 |
| D23 | 8/10 |
| D24 | 2/10 |
| D25 | 0/10 |
| D26 | 10/10 |
| D27 | 8/10 |
| D28 | 0/10 |
| L5 | 0/10 |

[a] Administered subcutaneously; 30 mg/kg × 2
[b] Compound numbers from Tables II and IV

TABLE XIII

Activity of C—20-Modified Derivatives Administered Orally to *Pasteurella multocida*-Infected Chicks[a]

| Test Compound[b] | Number of Deaths/Number Treated |
|---|---|
| D1 | 9/10 |
| D2 | 5/10 |
| D4 | 6/10 |
| D5 | 2/10 |
| D6 | 1/10 |
| D7 | 2/10 |
| D11 | 8/10 |
| D12 | 8/10 |
| D14 | 0/10 |
| D19 | 3/10 |
| D20 | 0/10 |
| D21 | 3/10 |
| D22 | 5/10 |
| D23 | 4/10 |
| D25 | 7/10 |
| D28 | 7/10 |

[a] Administered in the available drinking water at a concentration of 2 g/gal
[b] Compound numbers from Table II

This invention can be used to provide methods of controlling infections caused by bacterial and mycoplasmal species. In such methods, an effective amount of a compound of formula I is administered parenterally or orally to an infected or susceptible warm-blooded animal. The compounds can also be administered by insufflation, i.e. by blowing the compound, in the form of a medicated dust, into an enclosed space or room wherein the animals or poultry are held. The animals or poultry breathe the medicated dust present in the air; the medicated dust is also taken into the body through the eyes (a process called intraocular injection).

The dose which is effective to control the infection will vary with the severity of the infection and the age, weight, and condition of the animal. The total dose required for protection parenterally will generally, however, be in the range of from about 0.1 to about 100 mg/kg and preferably will be in the range of from about 0.5 to about 50 mg/kg. The dose required for oral administration will generally be in the range of from 1 to about 300 mg/kg and preferably will be in the range of from about 1 to about 100 mg/kg. Suitable dosage regimens can be constructed.

Often the most practical way to administer the compounds is by formulation into the feed supply or drinking water. A variety of feeds, including the common dry feeds, liquid feeds, and pelleted feeds, may be used.

In another aspect, this invention relates to compositions useful for the control of infections caused by bacteria and *Mycoplasma* species. These compositions comprise a compound of formula I together with a suitable vehicle. Compositions may be formulated for parenteral or oral administration by methods recognized in the pharmaceutical art.

The methods of formulating drugs into animal feeds are well-known. A preferred method is to make a concentrated-drug premix which in turn is used to prepare medicated feeds. Typical premixes may contain from about 1 to about 200 grams of drug per pound of premix. Premixes may be either liquid or solid preparations.

The final formulation of feeds for animals or poultry will depend upon the amount of drug to be administered. The common methods of formulating, mixing, and pelleting feeds may be used to prepare feeds containing a compound of formula I.

Effective injectable compositions containing these compounds may be in either suspension or solution form. In the preparation of suitable formulations it will be recognized that, in general, the water solubility of the acid addition salts is greater than that of the free bases. Similarly, the bases are more soluble in dilute acids or in acidic solutions than in neutral or basic solutions.

In the solution form the compound is dissolved in a physiologically acceptable vehicle. Such vehicles comprise a suitable solvent, preservatives such as benzyl alcohol, if needed, and buffers. Useful solvents include, for example, water and aqueous alcohols, glycols, and carbonate esters such as diethyl carbonate. Such aqueous solutions contain, in general, no more than 50% of the organic solvent by volume.

Injectable suspension compositions require a liquid suspending medium, with or without adjuvants, as a vehicle. The suspending medium can be, for example, aqueous polyvinylpyrrolidone, inert oils such as vegetable oils or highly refined mineral oils, or aqueous carboxymethylcellulose.

Suitable physiologically acceptable adjuvants are necessary to keep the compound suspended in suspension compositions. The adjuvants may be chosen from among thickeners such as carboxymethylcellulose, polyvinylpyrrolidone, gelatin, and the alginates. Many surfactants are also useful as suspending agents. Lecithin, alkylphenol polyethylene oxide adducts, naphthalenesulfonates, alkylbenzenesulfonates, and the polyoxyethylene sorbitan esters are useful suspending agents.

Many substances which affect the hydrophilicity, density, and surface tension of the liquid suspending medium can assist in making injectable suspensions in individual cases. For example, silicone antifoams, sorbitol, and sugars can be useful suspending agents.

In order to illustrate more fully the operation of this invention, the following examples are provided. In these examples the abbreviation "20-DH-DO" is used for the term "20-dihydro-20-deoxy".

Preparation 1

20-Dihydrotylosin (Relomycin)

A solution of tylosin base (30.0 g, 32.8 mmole) in 2-propanol (300 ml) and water (200 ml) was treated with sodium borohydride (315 mg, 8.2 mmole), portionwise, over a five-minute period. Thirty minutes after the addition was completed, the pH of the reaction solution was adjusted to 7.0 by the addition of 1N sulfuric acid solution. The neutralized solution was evaporated under vacuum to remove the 2-propanol; the aqueous solution remaining was treated with a saturated sodium bicarbonate solution (500 ml). The mixture was extracted with dichloromethane (3 × 300 ml), and the combined extracts were extracted with saturated sodium chloride solution (200 ml) and dried over sodium sulfate. Filtration followed by evaporation gave a glass which was broken up in *n*-hexane, collected on a filter and air-dried to yield 28.5 g (95%) of 20-dihydrotylosin.

Preparation 2

20-Dihydrodesmycosin

Desmycosin (10 g, 13 mmoles), dissolved in isopropanol:water (1:1, 175 ml), was stirred at room temperature while $NaBH_4$ (125 mg, 3.3 mmoles) was added. After 1/2 hour the pH of the reaction mixture was adjusted to 7.0 with 1*N* $H_2SO_4$. The alcohol was removed under reduced pressure. Saturated $NaHCO_3$ solution was added to the aqueous solution, and the product was extracted into $CH_2Cl_2$. The organic layer was dried ($Na_2SO_4$), and solvent was removed under reduced pressure to give 9.65 g of 20-dihydrodesmycosin (12.5 mmoles, 96% yield) as a white foam.

Preparation 3

20-DH-DO-20-Iododesmycosin *(Method 1)*

20-Dihydrodesmycosin (2.0 g, 2.6 mmoles) and tetra n-butylammonium iodide (1.5 g, 3.9 mmoles) were dissolved in $CH_2Cl_2$ (30 ml) with s-collidine (0.6 ml, 4.5 mmoles) added. This solution was cooled to −78°C under a nitrogen atmosphere and treated with trifluoromethanesulfonic anhydride (0.6 ml, 3.9 mmoles) dropwise by syringe. The reaction was stirred for 5 minutes at −78°C and then allowed to come to room temperature (about 30 minutes). Saturated $NaHCO_3$ solution was added, and the product was extracted with $CH_2Cl_2$. The organic layer was dried ($Na_2SO_4$) and evaporated to give a red oil which was purified by silica-gel flash chromatography, eluting initially with $CH_2Cl_2$ (400 ml) and then stepwise with $CH_2Cl_2$:$CH_3OH$ solutions as follows: 98:2 (250 ml); 96:4 (500 ml) 95:5 (250 ml); 94:6 (750 ml) and 92:8 (250 ml). Fractions containing the desired product were identified by TLC, combined and evaporated to dryness to give 20-DH-DO-20-iododesmycosin (595 mg, 0.67 mmoles, 26% yield) as a white foam.

Preparation 4

20-DH-DO-20-Iododesmycosin *(Method 2)*

20-Dihydrodesmycosin (5.0 g, 6.5 mmoles) and triphenylphosphine (2.54 g, 9.70 mmoles) were dissolved in dimethylformamide (DMF) (10 ml). This mixture was stirred at room temperature under $N_2$ while iodine (2.46 g, 9.70 mmoles) in DMF (5 ml) was added dropwise. The reaction mixture was stirred for two hours and then poured into cold saturated $NaHCO_3$ solution. The product was extracted with $CHCl_3$

(two portions), and the combined $CHCl_3$ extracts were shaken with 0.1 *M* sodium thiosulfate to remove unreacted iodine. The organic layer was dried ($Na_2SO_4$) and evaporated under reduced pressure to give a light yellow oil which was purified by silica-gel flash chromatography. The column was eluted initially with $CH_2Cl_2$ (500 ml) and then with 250 ml portions of $CH_2Cl_2$:$CH_3OH$ mixtures as follows: 98:2; 96:4; 95:5; 94:6; 92:8; 88:12; and 86:14. Fractions containing the desired product were identified as in Preparation 3 and combined to give 1.78 g (2.0 mmoles, 31% yield) of 20-DH-DO-20-iododesmycosin as a white foam.

Example 1

20-DH-DO-20-(Octahydroazocin-1-yl)desmycosin

20-DH-DO-20-Iododesmycosin (575 mg, 0.65 mmoles) was dissolved in acetonitrile (10 ml), and heptamethyleneimine (0.37 g, 0.41 ml, 3.3 mmoles) was added to this solution. The reaction was stirred at reflux for 1.5 hours. Volatiles were then removed under vacuum. The residue was dissolved in $CH_2Cl_2$ and extracted with saturated $NaHCO_3$ solution. The organic layer was dried ($Na_2SO_4$) and then evaporated under reduced pressure to give a light brown foam. This foam was purified by silica-gel flash chromatography, eluting with 250 ml each of the following $CH_2Cl_2$:$CH_3OH$ mixtures: 98:2; 96:4; 94:6; 9:1; 88:12; 82:18; 65:35; 1:1; 1:3 and finally with 300 ml of $CH_3OH$. Fractions containing the desired product were identified by TLC, combined and evaporated to dryness to give 397 mg (0.46 mmoles, 71% yield) of 20-DH-DO-20-(octahydroazocin-1-yl)desmycosin as a white foam.

Example 2

20-DH-DO-20-(Hexahydroazepin-1-yl)desmycosin

Desmycosin (10 g, 13 mmoles), dissolved in anhydrous methanol (100 ml), was added rapidly to a solution of $NaBH_3CN$ (3.3 g, 52 mmoles) and hexamethyleneimine (6.5 g, 7.5 ml, 65 mmoles) in anhydrous methanol (50 ml) under $N_2$. The reaction mixture was stirred under $N_2$ at room temperature for about three hours and then was evaporated under reduced pressure. The resultant residue was dissolved in $CH_2Cl_2$ with just enough ethyl acetate to aid in dissolving the residue, and this solution was extracted with saturated $NaHCO_3$ solution. The organic layer was separated, dried ($Na_2SO_4$), and evaporated under reduced pressure to give a light yellow foam. This foam was purified by silica-gel flash chromatography, eluting initially with $CH_2Cl_2$ (1 L), then stepwise with 500-ml portions of $CH_2Cl_2$:$CH_3OH$ mixtures as follows: 98:2; 96:4; 94:6; 92:8 and 9:1, and finally with $CH_2Cl_2$:$CH_3OH$:$NH_4OH$ mixtures as follows: 90:10:0.5 (500 ml) and 75:25:0.5 (2 L). Fractions containing the desired product were identified by TLC, combined and evaporated to dryness to give 6.035 g (7.07 mmoles) of 20-DH-DO-20-(hexahydroazepin-1-yl)desmycosin as a white foam. Other fractions which contained impure product were combined, redissolved in $CH_2Cl_2$, extracted again with saturated $NaHCO_3$ solution, and purifed as before, using a silica-gel column packed with $CH_2Cl_2$:$CH_3OH$ (9:1) and eluted with $CH_2Cl_2$:$CH_3OH$:$NH_4OH$ as follows: 90:10:0.5 (500 ml) and 80:20:0.5 (1 L) to give an additional 1.372 g (1.61 mmoles) of product. The total yield of 20-DH-DO-20-(hexahydroazepin-1-yl)desmycosin was 7.407 g (8.68 mmoles, 67%).

Example 3

20-DH-DO-20-(4-Phenylpiperidin-1-yl) desmycosin

Desmycosin (1.5 g, 2 mmole) was dissolved in absolute methanol (60 ml) and treated with 4-phenylpiperidine (640 mg, 4 mmoles) in the presence of Linde 4A molecular sieves. After 0.5 hr, $NaBH_3CN$ (500 mg, 8 mmoles) was added, and the mixture was stirred for 2.5 hr, at room temperature. The mixture was poured into saturated $NaHCO_3$ solution (200 ml) and extracted with $CH_2Cl_2$ (3 × 200 ml). The combined organic extracts were dried ($Na_2SO_4$), filtered and evaporated under reduced pressure. The residue (3.6 g) was purified by flash chromatography on silica gel, eluting with a gradient of 1 L. $CH_2Cl_2$ to 1 L. of MeOH:$CH_2Cl_2$ (5:95) and then with 1 L. of MeOH:$CH_2Cl_2$ (5:95). Fractions containing the desired product were located by TLC, combined and evaporated to dryness to yield 680 mg of 20-DH-DO-20-(4-phenylpiperidin-1-yl)desmycosin.

Example 4

20-DH-DO-20-(Hexahydroezepin-1-yl)-4′-deoxydesmycosin

A solution of 4′-deoxydesmycosin (565 mg, 0.75 mmole) in methanol (15 ml) under argon was stirred with activated Linde 3A molecular sieves (2.2 g) for thirty minutes before hexamethyleneimine (0.25 ml, 2.25 mmole) was added. One hour later, sodium cyanoborohydride (141 mg, 2.25 mmole) was added to the reaction. After an additional 45 minutes, the reaction mixture was poured into saturated sodium bicarbonate solution and extracted with ethyl acetate. The combined organic extracts were shaken with saturated sodium chloride solution, dried over sodium sulfate, filtered and evaporated to yield 600 mg of crude product. This product was purified by preparative TLC on silica gel, eluting with dichloromethane/methanol/conc. ammonium hydroxide (90:15:2) to give 150 mg (24% yield) of 20-DH-DO-20-(hexahydroezepin-1-yl)-4′-deoxydesmycosin.

Examples 5—6

20-DH-DO-20-(Octahydroazocin-1-yl)desmycosin, prepared by the method of Example 2.

20-DH-DO-20-(Hexahydroazepin-1-yl)desmycosin, prepared by the method of Example 1.

Example 7

20-DH-DO-20-(Octahydroazocin-1-yl)desmycosin *(Method 3)*

Desmycosin (4.0 g, 5.2 mmoles) was dissolved in absolute methanol (30 ml) and treated with heptamethyleneimine (1.2 g, 1.3 ml, 10.4 mmoles) in the presence of 3A molecular sieves. After the reaction mixture had been stirred for 1 hr at room temperature, a solution of $NaBH_4$ (60 mg, 1.6 mmoles) in absolute methanol (10 ml) was quickly added by pipette. The reaction mixture was stirred for 1.5 hr at room temperature, and then another 30 mg of $NaBH_4$ was added (one portion as the solid). The reaction mixture was stirred for another 75 min and then was filtered. The filtrate was evaporated under reduced pressure. The residue was dissolved in ethyl acetate (150 ml), and this solution was extracted with water (150 ml) and saturated $NaHCO_3$ solution (100 ml). The ethyl acetate solution was then extracted with pH 6.5, 0.5 *M* $NaH_2PO_4$ buffer (150 ml). The buffer extract was evaporated under vacuum to remove residual ethyl acetate and then was rapidly stirred while 5*N* NaOH was slowly added, yielding a thick white precipitate. The white solid was removed by filtration, washed with a small amount of water, and dried to give 3.55 g of 20-DH-DO-20-(octahydroazocin-1-yl)desmycosin.

Example 8

20-DH-DO-20-[1-Azaspiro[4.5]decan-1-yl]desmycosin

Desmycosin (5.0 g, 6.5 mmoles) was dissolved in absolute methanol (50 ml) and treated with 1-azaspiro[4.5]decane (1.36 g, 9.8 mmoles) in the presence of 3A molecular sieves. After 15 minutes, $NaBH_3CN$ (620 mg, 9.8 mmoles) was added, and the mixture was stirred for 17 hrs at room temperature. The reaction mixture was filtered, and the filtrate was evaporated under reduced pressure. The residue was dissolved in ethyl acetate (300 ml) and extracted with water (300 ml and 100 ml). The product was then extracted from the ethyl acetate solution with pH 6.5, 0.5 *M* $NaH_2PO_4$ buffer (300 ml and 100 ml). The phosphate buffer extracts were combined and evaporated under vacuum to remove residual ethyl acetate. The phosphate buffer solution was then rapidly stirred while 5*N* NaOH was slowly added, yielding a thick white precipitate. The white solid was removed by filtration, washed with water, and dried to give 20-DH-DO-20-[1-azaspiro[4.5]decan-1-yl]desmycosin (3.52 g).

Example 9

20-DH-DO-20-(1,2,3,4-Tetrahydroquinolin-1-yl)desmycosin

Desmycosin (11.6 g, 15 mmol) was dissolved in dry methanol (100 ml), and 1,2,3,4-tetrahydroquinoline (3.8 ml, 30 mmol) was added. After the mixture was stirred for 30 minutes at room temperature, sodium cyanoborohydride (1.25 g, 20 mmol) was added. The mixture was stirred overnight and then evaporated under reduced pressure. The residue was partitioned between ethyl acetate and water (100 ml each). The organic layer was then extracted sequentially with pH 6.5 aqueous phosphate buffer (100 ml) and pH 4.5 aqueous phosphate buffer (100 ml). The ethyl acetate layer was dried (sodium sulfate), filtered and evaporated; and the residue (4.6 g) was separated by chromatography on silica gel (Waters Prep 500). The column was eluted with a linear gradient of dichloromethane (4 L) and 5% methanol plus 0.5% conc. ammonium hydroxide in dichloromethane (4 L). Fractions containing the desired product were identified by TLC analysis, collected and evaporated to dryness to yield 3.4 g of the title compound.

Example 10

20-DH-DO-20-(1,2,3,4-Tetrahydroisoquinolin-2-yl)desmycosin

Desmycosin (11.6 g, 15 mmol) was dissolved in dry methanol (100 ml), and 1,2,3,4-tetrahydroisoquinoline (3.8 ml, 30 mmol) was added. After the mixture was stirred for 30 minutes at room temperature, sodium cyanoborohydride (1.25 g, 20 mmol) was added. The mixture was stirred overnight and then was evaporated under reduced pressure. The residue was partitioned between ethyl acetate and water (150 ml each). The organic layer was then extracted sequentially with pH 6.5 phosphate buffer (100 ml) and pH 4.5 phosphate buffer (100 ml). After evaporation of the pH 4.5 buffer extract under reduced pressure to remove ethyl acetate, the pH was adjusted to 10 with 5N sodium hydroxide. The precipitate which formed was collected and air-dried to yield 5.6 g of the title compound.

Example 11

20-DH-DO-20-(1,2,3,6-Tetrahydropyridin-1-yl)desmycosin

Desmycosin (11.6 g, 15 mmol) was dissolved in dry methanol (100 ml), and 1,2,3,6-tetrahydropyridine (2.8 ml, 30 mmol) was added. After the mixture was stirred for 30 minutes at room temperature, sodium cyanoborohydride (1.25 g, 20 mmol) was added. The mixture was stirred overnight and then was evaporated under reduced pressure. The residue was dissolved in ethyl acetate (150 ml). This solution was extracted with water (150 ml) and then with pH 6.5 aqueous phosphate buffer solution (2 × 100 ml). The buffer solutions were separately evaporated under reduced pressure to remove ethyl acetate and then adjusted to pH 10 with 5N sodium hydroxide. The precipitates which formed were collected by filtration and air-dried to yield 5.4 g (first extract) and 3.2 g (second extract) of the title compound.

Examples 12—31

The following compounds were prepared by the methods of Examples 1, 2, 7 or 8:

20-DH-DO-20-(octahydroazocin-1-yl)lactenocin
20-DH-DO-20-(pyrrolidin-1-yl)desmycosin
20-DH-DO-20-(azacyclotridecan-1-yl)desmycosin
20-DH-DO-20-(4-hydroxypiperidin-1-yl)desmycosin
20-DH-DO-20-(hexahydroazepin-1-yl)macrocin
20-DH-DO-20-[3-azabicyclo[3.2.2]nonan-3-yl]desmycosin
20-DH-DO-20-(piperidin-1-yl)desmycosin
20-DH-DO-20-[3-(N,N-diethylcarbamoyl)piperidin-1-yl]desmycosin
20-DH-DO-20-[(4-piperidino)piperidin-1-yl]desmycosin
20-DH-DO-20-(octahydro-1H-azonin-1-yl)desmycosin
20-DH-DO-20-(decahydroquinolin-1-yl)desmycosin
20-DH-DO-20-[1,3,3-trimethyl-6-azabicyclo[3.2.1]octan-6-yl]desmycosin
20-DH-DO-20-(dodecahydrocarbazol-9-yl)desmycosin
20-DH-DO-20-(octahydroazocin-1-yl)tylosin
20-DH-DO-20-(3-azabicyclo[3.2.2]nonan-3-yl)tylosin
20-DH-DO-20-(4-phenyl-1,2,3,6-tetrahydropyridin-1-yl)desmycosin
20-DH-DO-20-(4-benzyl-piperidine-1-yl)desmycosin
20-DH-DO-20-[4-(ethylenedioxy)-piperidin-1-yl]desmycosin
20-DH-DO-20-(octahydroazocin-1-yl)macrocin
20-DH-DO-20-(hexahydroazepin-1-yl)lactenocin

Examples 32—35

20-DH-DO-20-(3,3,5-Trimethylhexahydroazepin-1-yl)desmycosin was prepared by the method of Example 8 and then separated into individual isomers *1* and *2* silica-gel by flash chromatography.

20-DH-DO-20-(Dodecahydrocarbazol-9-yl)desmycosin (compound D25) was a mixture of two isomers. The mixture was separated into two fractions, each of which was rich in one of the isomers, by silica-gel flash chromatography. Each of the isomer-enriched fractions had an activity pattern similar to that of the mixture.

Examples 36—37

20-DH-DO-20-(Octahydroazocin-1-yl)desmycosin dihydrochloride and tartrate salts were prepared from 20-DH-DO-20-(octahydroazocin-1-yl)desmycosin, using standard procedures.

Tables XIV—XVI summarize certain physical data on exemplified compounds:

TABLE XIV
Physical Characteristics of C—20-Modified Derivatives of Desmycosin

| 20-Substituent | Compound[a] No. | FDMS Parent Ion $(m^+ + 1)$ | UV $\lambda max(\varepsilon)$[b] | Titratable groups[c] | TLC[d] $R_f$ |
|---|---|---|---|---|---|
| pyrrolidin-1-yl | D1 | 827 | | 7.9, 9.5 | 0.30 |
| 4-phenylpiperidin-1-yl | D4 | 917 | 283 (21,800) | | |
| hexahydroazepin-1-yl | D5 | 855 | 282 (21,500) | 7.9 9.6 | |
| hexahydroazepin-1-yl | D5a | 839 | 282 (21,500) | | |
| octahydroazocin-1-yl | D6 | 869 | 282 (21,750) | 7.9, 9.4 | |
| azocyclotridecan-1-yl | D10 | 939 | 282 (19,500) | 8.0, 9.5 | |
| 4-piperidinopiperidin-1-yl | D13 | 924 | 282 (18,600) | 6.0, 9.2 | |
| 3-azabicyclo[3.2.2]nonan-3-yl | D14 | 881 | 282 (20,500) | 7.9, 9.2 | |
| piperidin-1-yl | D2 | 841 | 282 (24,500) | 7.8, 9.1 | |
| 4-hydroxypiperidin-1-yl | D3 | 857 | 282 (20,750) | 7.1, 8.7 | |
| octahydro-1H-azonin-1-yl | D7 | 883 | 282 (20,000) | 7.9, 9.0 | |
| 1,2,3,4-tetrahydroquinolin-1-yl | D11 | 888 | 271 (26,700) 281 sh (24,500) | | |
| 1,2,3,4-tetrahydroisoquinolin-2-yl | D12 | 888 | 283 (20,600) | | |
| 3-(N,N-diethylcarbamoyl)piperidin-1-yl | D15 | 940 | 282 (20,500) | 7.4, 8.6 | |
| 1-azaspiro[4,5]decan-1-yl | D19 | 894 | 282 (21,500) | 7.9, 9.8 | |

TABLE XIV
Physical Characteristics of C—20-Modified Derivatives of Desmycosin (cont'd.)

| 20-Substituent | Compound[a] No. | FDMS Parent Ion ($m^+ + 1$) | UV $\lambda max(\varepsilon)$[b] | Titratable groups[c] | TLC[d] $R_f$ |
|---|---|---|---|---|---|
| decahydroquinolin-1-yl | D20 | 895 | 282 (21,500) | 7.9, 9.4 | |
| 1,3,3-trimethyl-6-azabicyclo[3.2.1]octan-1-yl | D21 | 908 | 282 (21,000) | 8.0, 9.7 | |
| 1,2,3,6-tetrahydropyridin-1-yl | D22 | 839 | 282 (21,000) | | |
| 3,3,5-trimethylhexahydroazepin-1-yl, isomer 1 | D23 | 896 | 282 (21,130) | 7.7, 9.0 | |
| 3,3,5-trimethylhexahydroazepin-1-yl, isomer 2 | D24 | 897 | 283 (21,390) | 7.9, 9.4 | |
| dodecahydrocarbazol-9-yl | D25 | 935 | 282 (22,500) | 7.95, 9.95 | |
| octahydroazocin-1-yl | D6[e] | | | 7.9, 9.4 | |
| octahydroazocin-1-yl | D6[f] | 869,151 | 282 (19,500) | 5.4, 7.3, | 9.2 |

[a] Compound numbers from Table II
[b] Run in methanol or ethanol
[c] Run in 66% aqueous DMF
[d] Silica-gel 60 plates; $CH_2Cl_2/MeOH/NH_4OH$ (90:10:0.5) solvent
[e] dihydrochloride salt
[f] tartrate salt

TABLE XV

Physical Characteristics of C—20-Modified Derivatives of Macrocin

| 20-Substituent | Compound[a] No. | FDMS Parent Ion ($m^+ + 1$) | UV λmax(ε)[b] | Titratable groups[c] | TLC[d] $R_f$ |
|---|---|---|---|---|---|
| hexahydroazepin-1-yl | M3 | 985 | | 6.91, 9.40 | 0.26 |
| octahydroazocin-1-yl | M4 | 999 | 282 (24,000) | 6.90, 9.25 | |

[a] Compound numbers from Table III
[b] Run in methanol
[c] Run in 66% aqueous DMF
[d] silica-gel 60 plates: $CH_2Cl_2$/MeOH/$NH_4OH$ (90:10:0.5) solvent

TABLE XVI
Physical Characteristics of C—20-Modified Derivatives of Lactenocin

| 20-Substituent | Compound[a] No. | FDMS Parent Ion ($m^+ + 1$) | UV λmax(ε) | Titratable groups[d] |
|---|---|---|---|---|
| hexahydroazepin-1-yl | L4 | 841 | 282 (21,500)[b] | 8.0, 9.70 |
| octahydroazocin-1-yl | L5 | 855 | 282 (21,500)[c] | 7.8, 9.3 |

[a] Compound numbers from Table IV
[b] Run in ethanol
[c] Run in methanol
[d] Run in 66% aqueous DMF

TABLE XVII
Physical Characteristics of C—20-Modified Derivatives of Tylosin

| 20-Substituent | Compound No. | FDMS Parent Ion ($m^+ + 1$) | UV λmax(ε) |
|---|---|---|---|
| octahydroazocin-1-yl | T6 | 1013 | 282 (22,000) |
| 3-azabicyclo[3.2.2]nonan-3-yl | T13 | 1025 | 282 (21,500) |

Examples 38—61

The following compounds can be prepared by the methods of the preceeding examples.

20-DH-DO-20-(octahydroazocin-1-yl)tylosin
20-DH-DO-20-(piperidin-1-yl)lactenocin
20-DH-DO-20-(4-hydroxypiperidin-1-yl)DOML
20-DH-DO-20-(decahydroazecin-1-yl)desmycosin
20-DH-DO-20-(octahydroazocin-1-yl)macrocin
20-DH-DO-20-(azacyclotridecan-1-yl)lactenocin
20-DH-DO-20-(hexahydroazepin-1-yl)lactenocin
20-DH-DO-20-(1,2,3,4-tetrahydroisoquinolin-2-yl)macrocin
20-DH-DO-20-(1,2,3,4-tetrahydroquinolin-1-yl)macrocin
20-DH-DO-20-(azacycloundecan-1-yl)desmycosin
20-DH-DO-20-(4-methylpiperidin-1-yl)desmycosin
20-DH-DO-20-(pyrrolidin-1-yl)lactenocin
20-DH-DO-20-(octahydro-1H-azonin-1-yl)tylosin
20-DH-DO-20-(octahydroazocin-1-yl)DOMM
20-DH-DO-20-(octahydroazocin-1-yl)DOML
20-DH-DO-20-(4-phenyl-piperidin-1-yl)lactenocin
20-DH-DO-20-(4-phenylpiperidin-1-yl)-4′-deoxydesmycosin
20-DH-DO-20-(octahydroazocin-1-yl)-4′-deoxydesmycosin
20-DH-DO-20-(3-azabicyclo[3.2.2]nonan-3-yl)-4′-deoxydesmycosin
20-DH-DO-20-(1,2,3,4-tetrahydroisoquinolin-2-yl)lactenocin
20-DH-DO-20-(3,3,5-trimethylhexahydroazepin-1-yl)macrocin
20-DH-DO-20-(decahydrocyclopent[c]azepin-1-yl)desmycosin
20-DH-DO-20-(7-azabicyclo[2.1.1]heptan-7-yl)desmycosin
20-DH-DO-20-(decahydroisoquinolin-2-yl)desmycosin

Example 62

*Injectable Formulations*

A) A formula I base is added to propylene glycol. Water and benzyl alcohol are added so that the solution contains 50% (by volume) propylene glycol, 4% (by volume) benzyl alcohol, and 200 mg/ml of a formula I base.

B) A solution is prepared as described in Section A except that the solution contains 50 mg/ml of a formula I base.

C) A solution is prepared as described in Section A except that the solution contains 350 mg/ml of a formula I base.

D) A solution is prepared as described in Section A except that the solution contains 500 mg/ml of a formula I tartrate.

E) A suspension is prepared by adding a finely ground formula I compound to carboxymethyl cellulose with thorough mixing so that the suspension contains 200 mg of the formula I base per ml of suspension.

Example 63

Chick Ration for Control of *Mycoplasma*

A balanced, high-energy ration adapted to feed chicks for rapid weight gain is prepared by the following recipe:

| Ingredient | % | Kg | (lbs) |
|---|---|---|---|
| Ground yellow corn | 50 | 454 | (1,000) |
| Soybean meal, solvent-extracted dehulled, finely ground, 50 percent protein | 31.09 | 282.0 | (621.8) |
| Animal fat (beef tallow) | 6.5 | 59 | (130) |
| Dried fish meal, with solubles (60% protein) | 5.0 | 45.4 | (100) |
| Distillers' solubles from corn | 4.0 | 36 | (80) |
| Dicalcium phosphate, feed grade | 1.8 | 16 | (36) |
| Calcium carbonate | 0.8 | 7.3 | (16) |
| Vitamin premix (representing vitamins A, D, E, K, and $B_{12}$, choline, niacin, pantothenic acid, riboflavin, biotin, with glucose bulking agent) | 0.5 | 4.5 | (10) |
| Trace mineral premix (representing $MnSO_4$, ZnO, KI, $FeSO_4$, $CaCO_3$) | 0.2 | 1.8 | (4) |
| 2-Amino-4-hydroxybutyric acid (hydroxy analog of methionine) | 0.1 | 0.9 | (2) |
| Formula *1* or *2* compound | 0.01 | 0.09 | (0.2) |

These substances are mixed in accordance with standard feed-mixing techniques. Chicks fed such a ration, with water *ad libitum*, are protected against exposure to *Mycoplasma* infections.

**Claims**

1. A macrolide of the formula (I):

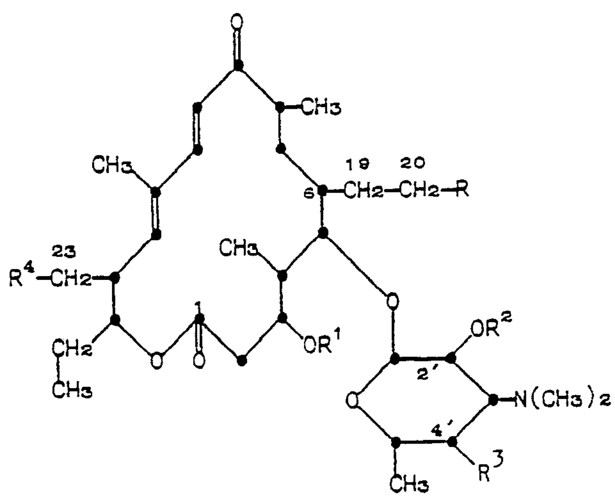

wherein

R is a monocyclic saturated or unsaturated ring containing one nitrogen atom as a sole heteroatom, the ring being bonded through the nitrogen atom and including from 5 to 16 ring atoms, or a bicyclic or tricyclic saturated or unsaturated ring containing one nitrogen atom as the sole heteroatom, the ring being bonded through the nitrogen atom and including from 8 to 20 ring atoms, the monocyclic, bicyclic, or tricyclic ring being optionally substituted at one or more of the carbon atoms by

$C_1$—$C_4$ alkyl,
hydroxyl,
di-($C_1$—$C_4$ alkyl)amino,

$-\overset{\frown}{N(CH_2)_m}$, where m = 4—7,

$-\overset{\overset{O}{\|}}{C}N(C_1-C_4\ alkyl)_2$,

$-\overset{\overset{O}{\|}}{C}N(CH_2)_m$, where m = 4—7,

$C_1-C_4$ alkoxycarbonyl,
phenyl,
phenyl substituted by 1, 2, or 3 groups selected from nitro, halo, $C_1-C_4$ alkyl, $C_1-C_4$ alkoxy, hydroxy, amino, and mono- or di=($C_1-C_4$ alkyl)amino,
benzyl,
$C_2-C_4$ alkenyl,
$C_2-C_4$ alkynyl,
$C_1-C_4$ alkoxy,
$C_1-C_4$ alkanoyloxy,
halo,
halo-($C_1-C_4$ alkyl)-,
cyano,
ethylenedioxy;

$R^1$ is hydrogen or a hydroxyl protecting group;

$R^2$ is hydrogen, optionally substituted $C_1-C_5$-alkanoyl, optionally substituted phenylacetyl, optionally substituted phenylpropionyl, or optionally substituted benzoyl;

$R^3$ is hydrogen, iodo, hydroxy, optionally substituted $C_1-C_5$-alkanoyloxy, optionally substituted benzoyloxy, optionally substituted phenylacetoxy, optionally substituted phenoxyacetoxy, or

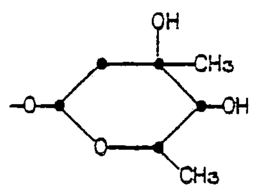

(mycarosyloxy)

$R^4$ is

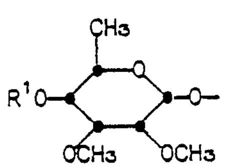

;

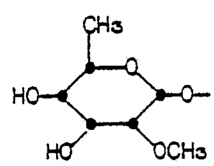

; or

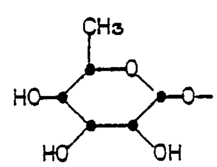

the group of formula $R^4$ having the same conformation as the mycinosyloxy group of tylosin, wherein $R^1$ is as defined above, and the acid addition salts thereof, provided that if $R^3$ is hydrogen or iodo then $R^4$ is

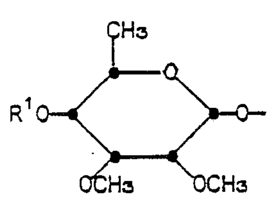

2. A macrolide of formula (I) in accordance with claim 1 wherein R is

(i) a saturated monocyclic ring of the formula $-N(CH_2)_n$, where n is an integer from 4 through 15, which ring is optionally substituted at one or more of the carbon atoms by $C_1$—$C_3$ alkyl, hydroxy, $-N(R^6)_2$, where $R^6$ is methyl, ethyl, n-propyl, isopropyl or the $R^6$ groups taken together with the nitrogen atom form pyrrolidinyl, piperidinyl, hexahydroazepinyl, or octahydroazocinyl,

$$-\overset{\displaystyle O}{\overset{\|}{C}}-N(R^6)_2,$$

where $R^6$ is as defined above, carbomethoxy, carboethoxy, or phenyl, or

(ii) a bicyclic or tricyclic secondary amino group selected from 1,2,3,4-tetrahydroquinolin-1-yl, decahydroquinolin-1-yl, 1,2,3,4-tetrahydroisoquinolin-2-yl, decahydroisoquinolin-2-yl, indolin-1-yl, isoindolin-2-yl, decahydrocyclohepta[b]pyrrol-1-yl, decahydrocyclohepta[c]pyrrol-2-yl, decahydrocyclopent-[c]azepin-2-yl, decahydrocyclopent[d]azepin-3-yl, 2,3,4,5-tetrahydro-1H-2-benzazepin-2-yl, 2,3,4,5-tetrahydro-1H-3-benzazepin-3-yl, azabicycloheptanyl, azabicyclooctanyl, azabicyclononanyl, azabicyclodecanyl, or azatricyclodecanyl.

3. A macrolide of formula (I) or a pharmaceutically-acceptable salt thereof as claimed in claim 1, wherein R represents a group selected from octahydroazocin-1-yl, hexahydroazepin-1-yl, 4-phenylpiperidin-1-yl, pyrrolidin-1-yl, azocyclotridecan-1-yl, 4-hydroxypiperidin-1-yl, 3-azabicyclo[3.2.2.]nonan-3-yl, piperidin-1-yl, 3-(N,N-diethylcarbamoyl)piperidin-1-yl, (4-piperidino)piperidin-1-yl, octahydro-1H-azocin-1-yl, decahydroquinolin-1-yl, 1,2,3,4-tetrahydroquinolin-1-yl, 1,2,3,4-tetrahydroisoquinolin-2-yl, 1,3,3-trimethyl-6-azabicyclo[3.2.1.]octan-6-yl, 1-azaspiro[4.5]decan-1-yl, 1,2,3,6-tetrahydro-pyridin-1-yl, dodecahydrocarbazol-9-yl, 3,3,5-trimethylhexahydroazepin-1-yl, decahydroazecin-1-yl, azacyclotridecan-1-yl, azacycloundecan-1-yl, 4-methylpiperidin-1-yl, 4-phenyl-piperidin-1-yl, decahydrocyclopent[c]azepin-1-yl, 7-azabicyclo[2.2.1]heptan-1-yl.

4. A macrolide of formula (I), or a pharmaceutically-acceptable salt thereof, as claimed in any one of claims 1 to 3, in which $R^4$ is mycinosyloxy and $R^3$ is hydroxyl.

5. A macrolide of formula (I) as claimed in any one of claims 1—4 wherein R is a saturated monocyclic ring.

6. 20-DH-20-DO-[3-azabicyclo[3.2.2.]nonan-3-yl]-desmycosin.

7. A process for preparing a macrolide of formula (I) or a pharmaceutically-acceptable salt thereof, as claimed in any one of claims 1—6, which comprises

(a) reducing an aldehyde of formula (III)

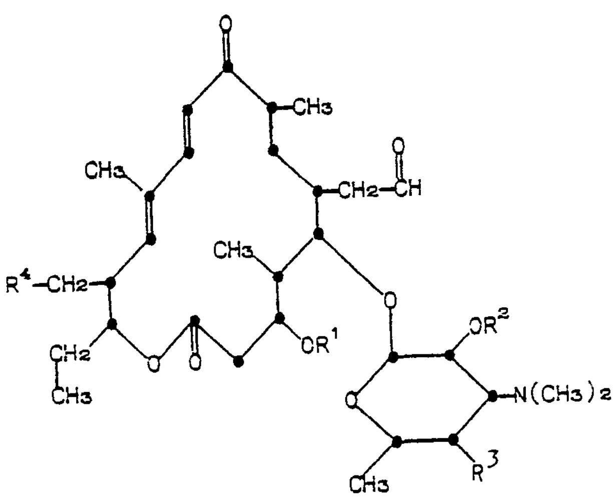

where $R^1$, $R^2$, $R^3$ and $R^4$ are as defined in formula (I) in the presence of an amine of formula HR where R is as defined in formula (I), or

(b) reacting an amine of formula HR, where R is as defined in formula (I) with a macrolide of formula (IV)

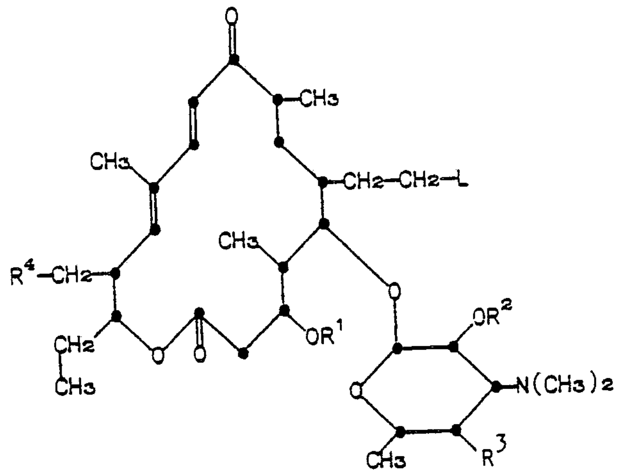

wherein L is a leaving group capable of being displaced by the amine HR, in a nonreactive organic solvent, or

(c) cleaving the mycarose sugar from a macrolide of formula (I) in which $R^3$ is mycarosyloxy by acid hydrolysis to provide a macrolide of formula (I) wherein $R^3$ is hydroxy, or

(d) converting a sulfonic ester of the formula (V)

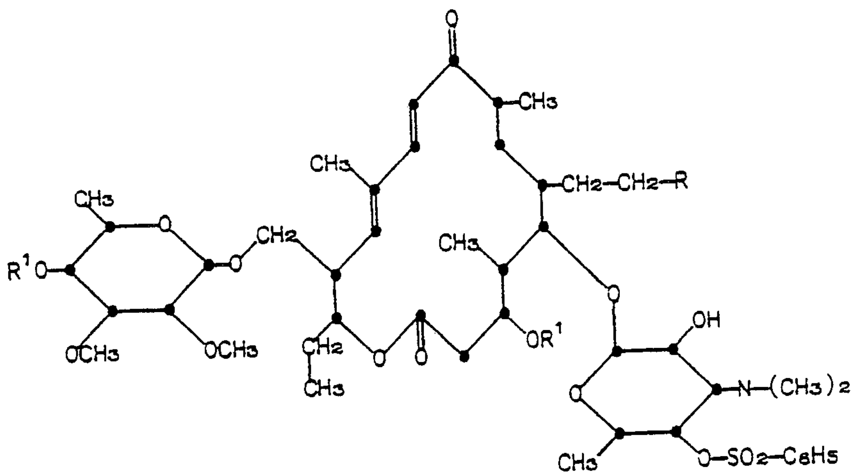

wherein R and $R^1$ are as defined in formula (I) to the corresponding 4'-iodide by reacting the sulfonic ester with a source of iodide ion in an inert organic solvent, and if $R^1$ is other than hydrogen, optionally hydrolyzing to provide the corresponding macrolide wherein $R^1$ is hydrogen or

(e) reductively deiodinating a macrolide of formula (VI)

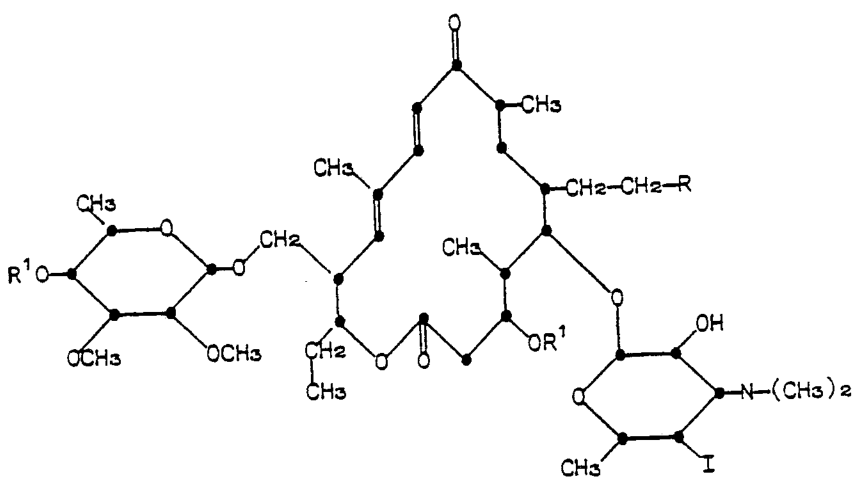

wherein R is as defined in formula (I) and each $R^1$ is a hydroxyl protecting group to produce a macrolide of formula (I) wherein $R^3$ is H, and optionally removing the hydroxyl protecting groups to provide a macrolide of formula (I) wherein $R^1$ and $R^3$ are hydrogen, and

(f) if necessary, esterifying or salifying, or both, a product of reaction (a), (b), (c), (d), or (e).

8. A macrolide of formula (I) or a pharmaceutically-acceptable salt thereof as claimed in any one of claims 1 to 6 for use as an antibiotic in the chemotherapy of warm blooded animals.

9. A feed premix comprising as an active ingredient a macrolide of formula (I), or a pharmaceutically-acceptable salt thereof as claimed in any one of claims 1—6.

10. A veterinary formulation which comprises as an active ingredient a macrolide of formula (I) or a pharmaceutically-acceptable salt thereof, as claimed in any one of claims 1—6, associated with one or more physiologically-acceptable carriers or vehicles therefor.

**Patentansprüche**

1. Makrolid der Formel (I)

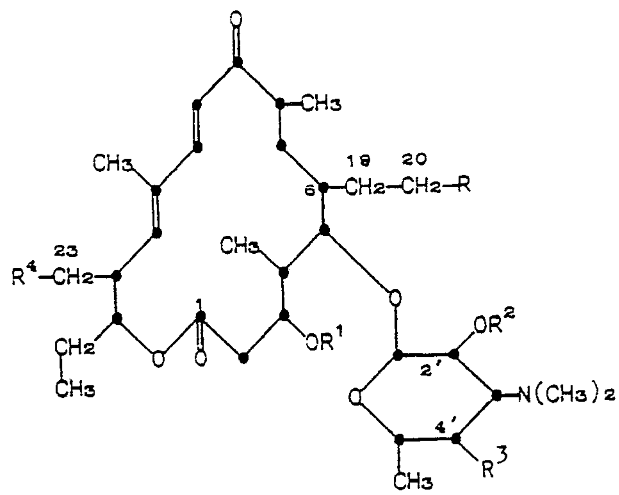

worin

R einen monocyclischen gesättigten oder ungesättigten Ring, der ein Stickstoffatom als einziges Heteroatom enthält, wobei dieser Ring über das Stickstoffatom gebunden ist und 5 bis 16 Ringatome aufweist, oder einen bicyclischen oder tricyclischen gesättigten oder ungesättigten Ring bedeutet, der ein Stickstoffatom als einziges Heteroatom enthält, wobei dieser Ring über das Stickstoffatom gebunden ist und 8 bis 20 Ringatome aufweist, wobei der monocyclische, bicyclische oder tricyclische Ring gegebenenfalls an einem oder mehreren seiner Kohlenstoffatome substituiert ist durch

$C_1$—$C_4$-Alkyl,
Hydroxy,
Di-($C_1$—$C_4$-alkyl)amino,

$-N(CH_2)_m$, worin m für 4 bis 7 steht,

$-\overset{O}{\overset{\|}{C}}N-(C_1-C_4\text{-Alkyl})_2$,

$-\overset{O}{\overset{\|}{C}}N(CH_2)_m$, worin m für 4 bis 7 steht,
$C_1$—$C_4$-Alkoxycarbonyl,
Phenyl,
Phenyl, das substituiert ist durch eine, zwei oder drei Gruppen, ausgewählt aus Nitro, Halogen, $C_1$—$C_4$-Alkyl, $C_1$—$C_4$-Alkoxy, Hydroxy, Amino und Mono- oder Di-($C_1$—$C_4$-alkyl)-amino,
Benzyl,
$C_2$—$C_4$-Alkenyl,
$C_2$—$C_4$-Alkinyl,
$C_1$—$C_4$-Alkoxy,

$C_1$—$C_4$-Alkanoyloxy,
Halogen,
Halogen-($C_1$—$C_4$-alkyl),
Cyano oder
Ethylendioxy,

$R^1$ Wasserstoff oder eine Hydroxyschutzgruppe ist,

$R^2$ für Wasserstoff, gegebenenfalls substituiertes $C_1$—$C_5$-Alkanoyl, gegebenenfalls substituiertes Phenylacetyl, gegebenenfalls substituiertes Phenylpropionyl oder gegebenenfalls substituiertes Benzoyl steht,

$R^3$ Wasserstoff, Iod, Hydroxy, gegebenenfalls substituiertes $C_1$—$C_5$-Alkanoyloxy, gegebenenfalls substituiertes Benzoyloxy, gegebenenfalls substituiertes Phenylacetoxy, gegebenenfalls substituiertes Phenoxyacetoxy oder

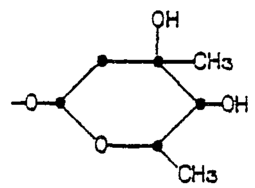

(mycarosyloxy)

bedeutet und

$R^4$ für

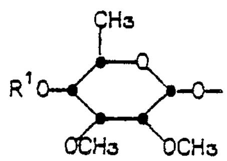 ; 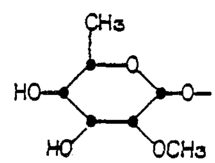 ; oder 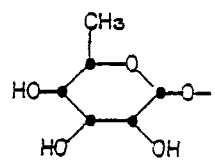

steht, wobei die Gruppe der Formel $R^4$ die gleiche Konformation hat wie die Mycinosyloxygruppe von Tylosin und worin $R^1$ die oben angegebenen Bedeutungen hat, und die Säureadditionssalze hiervon, wobei $R^4$ jedoch für

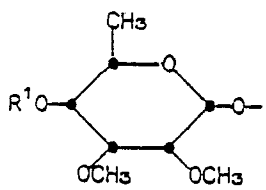

steht, falls $R^3$ Wasserstoff oder Iod ist.

2. Makrolid der Formel (I) nach Anspruch 1, dadurch gekennzeichnet, daß R folgende Bedeutungen hat:

(I) ein gesättigter monocyclischer Ring der Formel $—N(CH_2)_n$, worin n eine Zahl von 4 bis 15 ist und der gegebenenfalls an einem oder mehreren seiner Kohlenstoffatome substituiert ist durch $C_1$—$C_3$-Alkyl, Hydroxy, —$N(R^6)_2$, worin $R^6$ Methyl, Ethyl, n-Propyl oder Isopropyl ist oder die Gruppen $R^6$ zusammen mit dem Stickstoffatom Pyrrolidinyl, Piperidinyl, Hexahydroazepinyl oder Octahydroazocinyl bilden,

$$—\overset{\overset{O}{\|}}{C}—N(R^6)_2,$$

worin $R^6$ wie oben definiert ist, Carbomethoxy, Carbethoxy oder Phenyl, oder

(II) eine bicyclische oder tricyclische sekundäre Aminogruppe, die ausgewählt ist aus 1,2,3,4-Tetrahydrochinolin-1-yl, Decahydrochinolin-1-yl, 1,2,3,4-Tetrahydroisochinolin-2-yl, Decahydroisochinolin-2-yl, Indolin-1-yl, Isoindolin-2-yl, Decahydrocyclohepta[b]pyrrol-1-yl, Decahydrocyclohepta[c]pyrrol-2-yl, Decahydrocyclopent[c]azepin-2-yl, Decahydrocyclopent[d]azepin-3-yl, 2,3,4,5-Tetrahydro-1H-2-benzazepin-2-yl, 2,3,4,5-Tetrahydro-1H-3-benzazepin-3-yl, Azabicycloheptanyl, Azabicyclooctanyl, Azabicyclononanyl, Azabicyclodecanyl oder Azatricyclodecanyl.

3. Makrolid der Formel (I) oder ein pharmazeutisch annehmbares Salz hiervon nach Anspruch 1, dadurch gekennzeichnet, daß R eine Gruppe bedeutet, die ausgewählt ist aus Octahydroazocin-1-yl, Hexa-

hydroazepin-1-yl, 4-Phenylpiperidin-1-yl, Pyrrolidin-1-yl, Azocyclotridecan-1-yl, 4-Hydroxypiperidin-1-yl, 3-Azabicyclo[3.2.2.]nonan-3-yl, Piperidin-1-yl, 3-(N,N-Diethylcarbamoyl)piperidin-1-yl, (4-Piperidino)-piperidin-1-yl, Octahydro-1H-azocin-1-yl, Decahydrochinolin-1-yl, 1,2,3,4-Tetrahydrochinolin-1-yl, 1,2,3,4-Tetrahydroisochinolin-2-yl, 1,3,3-Trimethyl-6-azabicyclo[3.2.1]octan-6-yl, 1-Azaspiro[4.5]decan-1-yl, 1,2,3,6-Tetrahydropyridin-1-yl, Dodecahydrocarbazol-9-yl, 3,3,5-Trimethylhexahydroazepin-1-yl, Decahydroazecin-1-yl, Azacyclotridecan-1-yl, Azacycloundecan-1-yl, 4-Methylpiperidin-1-yl, 4-Phenylpiperidin-1-yl, Decahydrocyclopent[c]azepin-1-yl oder 7-Azabicyclo[2.2.1]heptan-1-yl.

4. Makrolid der Formel (I) oder ein pharmazeutisch annehmbares Salz hiervon nach irgendeinem der Ansprüche 1 bis 3, dadurch gekennzeichnet, daß $R^4$ Mycinosyloxy ist und $R^3$ Hydroxy bedeutet.

5. Makrolid der Formel (I) nach irgendeinem der Ansprüche 1 bis 4, dadurch gekennzeichnet, daß R ein gesättigter monocyclischer Ring ist.

6. 20-DH-20-DO-[3-Azabicyclo[3.2.2.]nonan-3-yl]desmycosin.

7. Verfahren zur Herstellung eines Makrolids der Formel (I) oder eines pharmazeutisch annehmbaren Salzes hiervon nach irgendeinem der Ansprüche 1 bis 6, dadurch gekennzeichnet, daß man

(a) eine Aldehyd der Formel (III)

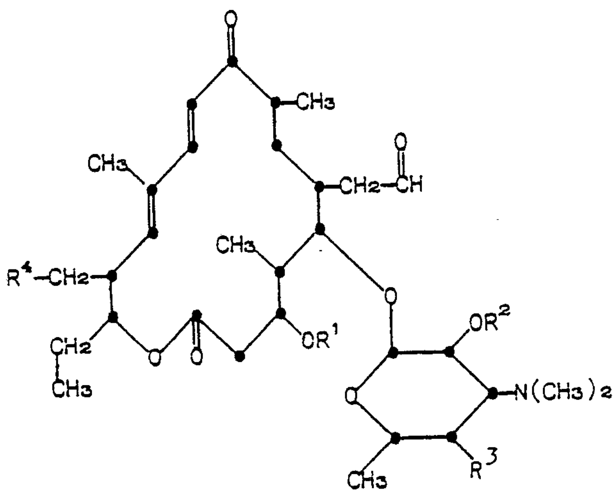

worin $R^1$, $R^2$, $R^3$ und $R^4$ wie in der Formel (I) definiert sind, in Gegenwart eines Amins der Formel HR, worin R wie in der Formel (I) definiert ist, reduziert oder

(b) ein Amin der Formel HR, worin R wie in der Formel (I) definiert ist, mit einem Makrolid der Formel (IV)

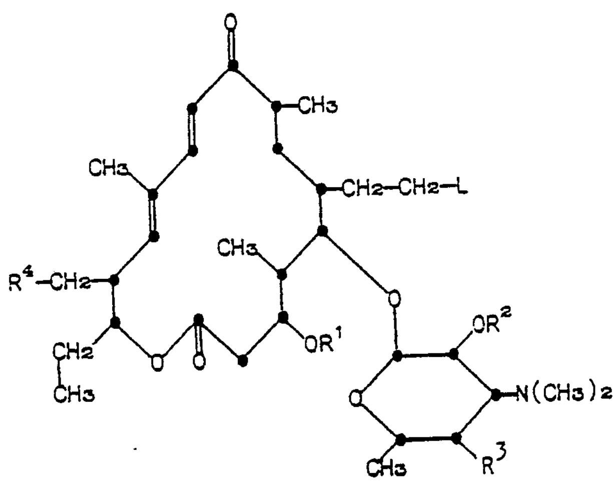

worin L eine Abgangsgruppe bedeutet, die durch das Amin HR ausgetauscht werden kann, in einem nichtreaktionsfähigen organischen Lösungsmittel umsetzt oder

(c) den Mycarosezucker von einem Makrolid der Formel (I), worin $R^3$ für Mycarosyloxy steht, durch saure Hydrolyse abspaltet und so ein Makrolid der Formel (I) bildet, worin $R^3$ Hydroxy ist, oder

(d) einen Sulfonsäureester der Formel (V)

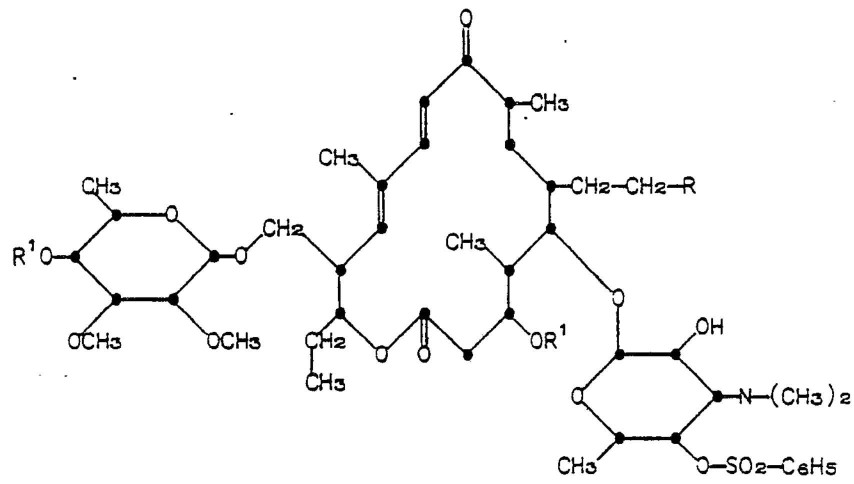

worin R und $R^1$ wie in der Formel (I) definiert sind, durch Umsetzung des Sulfonsäureesters mit einer Quelle für ein Iodidion in einem inerten organischen Lösungsmittel in das entsprechende 4'-Iodid überführt und die erhaltene Verbindung, falls $R^1$ eine andere Bedeutung als Wasserstoff hat, gegebenenfalls hydrolysiert und so das entsprechende Makrolid bildet, worin $R^1$ Wasserstoff ist, oder

(e) ein Makrolid der Formel (VI)

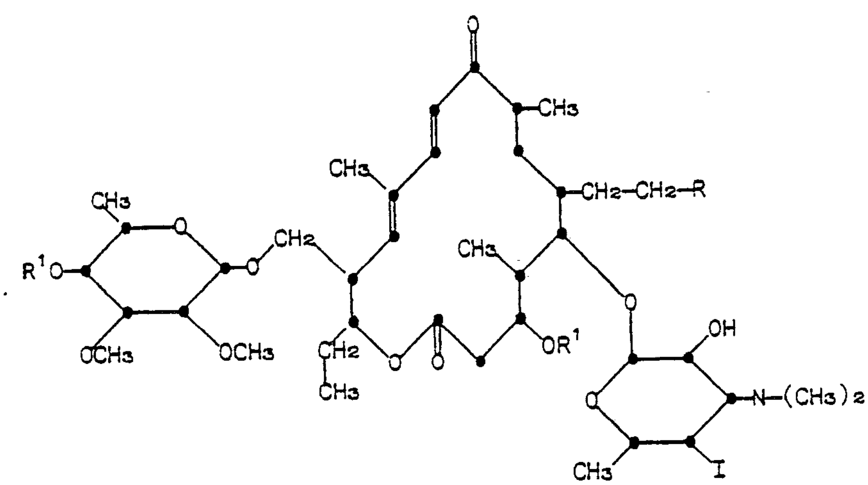

worin R wie in der Formel (I) definiert ist und jeder der Substituenten $R^1$ eine Hydroxyschutzgruppe ist, durch reduktive Deiodierung in ein Makrolid der Formel (I) überführt, worin $R^3$ für H steht, und von der erhaltenen Verbindung gegebenenfalls die Hydroxyschutzgruppen entfernt und so ein Makrolid der Formel (I) bildet, worin $R^1$ und $R^3$ jeweils Wasserstoff sind, und

(f) ein gemäß Umsetzung (a), (b), (c), (d) oder (e) erhaltenes Produkt erforderlichenfalls verestert und/oder in ein Salz überführt.

8. Verwendung eines Makrolids der Formel (I) oder eines pharmazeutisch annehmbaren Salzes hiervon nach irgendeinem der Ansprüche 1 bis 6 als Antibiotikum bei der Chemotherapie warmblütiger Tiere.

9. Futtervormischung dadurch gekennzeichnet, daß sie als Wirkstoff ein Makrolid der Formel (I) oder ein pharmazeutisch annehmbares Salz hiervon nach irgendeinem der Ansprüche 1 bis 6 enthält.

10. Veterinärmedizinische Formulierung, dadurch gekennzeichnet, daß sie ein Makrolid der Formel (I) oder ein pharmazeutisch annehmbares Salz hiervon nach irgendeinem der Ansprüche 1 bis 6 als Wirkstoff in Verbindung mit einem oder mehreren physiologisch annehmbaren Trägern oder Hilfsmitteln hierfür enthält.

**Revendications**

1. Macrolide de formule (I):

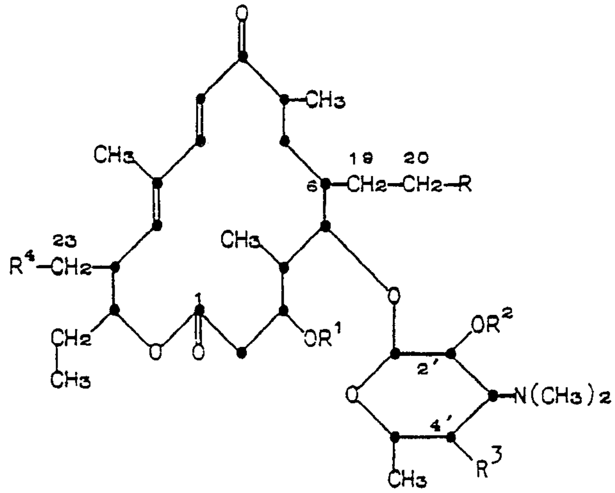

dans laquelle

R représente un noyau monocyclique saturé ou insaturé contenant 1 atome d'azote comme unique hétéro-atome, ce noyau étant lié par l'atome d'azote et comprenant 5 à 16 atomes cycliques, ou un noyau bicyclique ou tricyclique saturé ou insaturé contenant un atome d'azote comme unique hétéro-atome, ce noyau étant lié par l'atome d'azote et contenant 8 à 20 atomes cycliques, le noyau monocyclique, bicyclique ou tricyclique étant éventuellement substitué sur un ou plusieurs des atomes de carbone par:

un groupe alkyle en $C_1$—$C_4$,
un groupe hydroxy,
un groupe di-(alkyl en $C_1$—$C_4$)amino,

un groupe $-\widehat{N(CH_2)_m}$ où m = 4—7,

un groupe $-\overset{\overset{O}{\|}}{C}N(\text{alkyle en } C_1-C_4)_2$,

un groupe $-\overset{\overset{O}{\|}}{C}\widehat{N(CH_2)_m}$ où m = 4—7,

un groupe alcoxy(en $C_1$—$C_4$)carbonyle,
un groupe phényle,
un groupe phényle substitué par 1, 2 ou 3 groupes choisis parmi le groupe nitro, les atomes d'halogènes, les groupes alkyle en $C_1$—$C_4$, les groupes alcoxy en $C_1$—$C_4$, le groupe hydroxy, le groupe amino et les groupes mono- ou di-(alkyl en $C_1$—$C_4$)amino,
le groupe benzyle,
les groupes alcényle en $C_2$—$C_4$,
les groupes alcynyle en $C_2$—$C_4$,
les groupes alcoxyl en $C_1$—$C_4$,
les groupes alcanoyl(en $C_1$—$C_4$)oxy,
les atomes d'halogènes,
les groupes halo-(alkyle en $C_1$—$C_4$),
le groupe cyano,
le groupe éthylène-dioxy;

$R^1$ représente un atome d'hydrogène ou un groupe protectuer du groupe hydroxy;

$R^2$ représente un atome d'hydrogène, un groupe alcanoyle en $C_1$—$C_5$ éventuellement substitué, un groupe phénylacétyle éventuellement substitué, un groupe phénylpropionyl éventuellement substitué ou un groupe benzoyle éventuellement substitué;

$R^3$ représente un atome d'hydogène, un atome d'iode, un groupe hydroxy, un groupe alcanoyl(en $C_1$—$C_5$)oxy éventuellement substitué, un groupe benzoyloxy éventuellement substitué, un groupe phénylacétoxy éventuellement substitué, un groupe phénoxyacétoxy éventuellement substitué ou

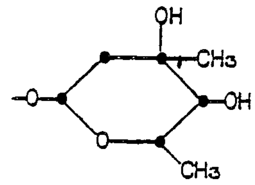

(mycarosyloxy)

$R^4$ représente

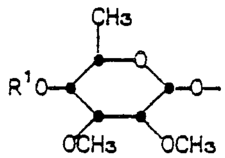 ; 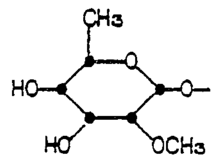 ; ou 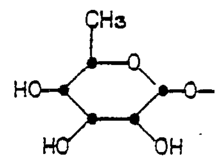

le groupe de formule $R^4$ ayant le même conformation que le groupe mycinosyloxy de la tylosin, $R^1$ ayant la signification définie ci-dessus, ainsi que ses sels d'addition d'acide, avec cette réserve que, si $R^3$ est un atome d'hydrogène ou un atome d'iode, $R^4$ représente

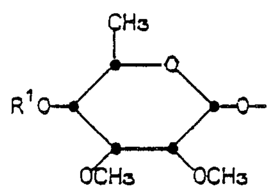

2. Macrolide de formule (I) selon la revendication 1, dans lequel R représente

(i) un noyau monocyclique saturé de formule $-N(CH_2)_n$ dans laquelle n est un nombre entier de 4 à 15, ce noyau étant éventuellement substitué sur un ou plusieurs des atomes de carbone par: un groupe alkyle en $C_1-C_3$, un groupe hydroxy, un groupe $-N(R^6)_2$ où $R^6$ représente un groupe méthyle, un groupe éthyle, un groupe n-propyle, un groupe isopropyle ou encore les groupes $R^6$ pris ensemble avec l'atome d'azote, forment un groupe pyrrolidinyle, un groupe pipéridinyl, un groupe hexahydroazépinyle ou un groupe octahydroazocinyle, un groupe

$$-\overset{\overset{\displaystyle O}{\|}}{C}-N(R^6)_2$$

où $R^6$ a la signification définie ci-dessus, un groupe carbométhoxy, un groupe carboéthoxy, ou un groupe phényle ou

(ii) un groupe amino secondaire bicyclique ou tricyclique choisi parmi: le groupe 1,2,3,4-tétrahydroquinolin-1-yle, le groupe décahydroquinolin-1-yle, le groupe 1,2,3,4-tétrahydroisoquinolin-2-yle, le groupe décahydroisoquinolin-2-yle, le groupe indolin-1-yle, le groupe isoindolin-2-yle, le groupe décahydrocyclohepta[b]pyrrol-1-yle, le groupe décahydrocyclohepta[c]pyrrol-2-yle, le groupe décahydrocyclopent[c]azépin-2-yle, le groupe décahydrocyclopent[d]azépin-3-yle, le groupe 2,3,4,5-tétrahydro-1H-2-benzazépin-2-yle, le groupe 2,3,4,5-tétrahydro-1H-3-benzazépin-3-yle, le groupe azabicycloheptanyle, le groupe azabicyclo-octanyle, le groupe azabicyclononanyle, le groupe azabicyclodécanyle, ou le groupe azatricyclodécanyle.

3. Macrolide de formule (I) ou un de ses sels pharmaceutiquement acceptables selon la revendication 1, caractérisé en ce que R représente un groupe choisi parmi le groupe octahydroazocin-1-yle, le groupe hexahydroazépin-1-yle, le groupe 4-phényl-pipéridin-1-yle, le groupe pyrrolidin-1-yle, le groupe azocyclotridécan-1-yle, le groupe 4-hydroxypipéridin-1-yle, le groupe 3-azabicyclo[3.2.2.]nonan-3-yle, le groupe pipéridin-1-yle, le groupe 3-(N,N-diéthylcarbamoyl)pipéridin-1-yle, le groupe (4-pipéridino)pipéridin-1-yle, le groupe octahydro-1H-azocin-1-yle, le groupe décahydroquinolin-1-yle, le groupe 1,2,3,4-tétrahydroquinolin-1-yle, le groupe 1,2,3,4-tétrahydroisoquinolin-2-yle, le groupe 1,3,3-triméthyl-6-azabicyclo-[3.2.1.]octan-6-yle, le groupe 1-azaspiro[4.5]décan-1-yle, le groupe 1,2,3,6-tétrahydro-pyridin-1-yle, le groupe dodécahydrocarbazol-9-yle, le groupe 3,3,5-triméthylhexahydroazépin-1-yle, le groupe décahydro-

azécin-1-yle, le groupe azacyclotridécan-1-yle, le groupe azacycloundécan-1-yle, le groupe 4-méthyl-pipéridin-1-yle, le groupe 4-phényl-pipéridin-1-yle, le groupe décahydrocyclopent[c]azépin-1-yle, le groupe 7-azabicyclo[2.2.1]heptan-1-yle.

4. Macrolide de formule (I) ou un de ses sels pharmaceutiquement acceptables selon l'une quelconque des revendications 1 à 3, caractérisé en ce que $R^4$ est un groupe mycinosyloxy et $R^3$ est un groupe hydroxy.

5. Macrolide de formule (I) selon l'une quelconque des revendications 1 à 4, caractérisé en ce que R est un noyau monocyclique saturé.

6. La 20-DH-20-DO-[3-azabicyclo[3.2.2.]nonan-3-yl]desmycosine.

7. Procédé de préparation d'un macrolide de formule (I) ou d'un de ses sels pharmaceutiquement acceptables selon l'une quelconque des revendications 1—6, caractérisé en ce qu'il comprend les étapes qui consistent à:

(a) réduire un aldéhyde de formule (III):

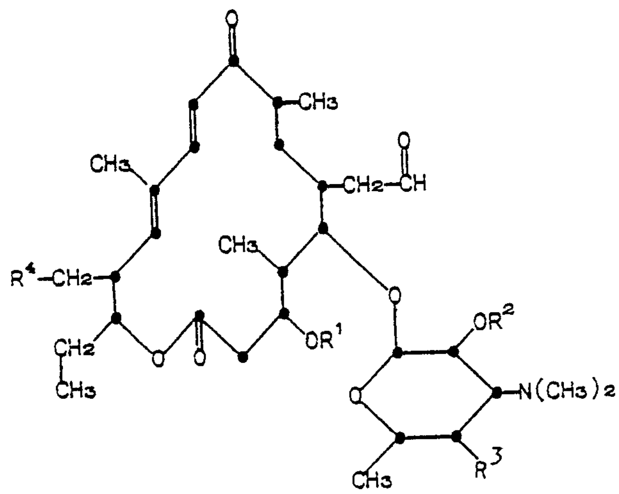

dans laquelle $R^1$, $R^2$, $R^3$ et $R^4$ ont les significations définies dans la formule (I) en présence d'une amine de formule HR dans laquelle R a la signification définie dans la formule (I), ou

(b) faire réagir une amine de formule HR dans laquelle R a la signification définie dans la formule (I), avec un macrolide de formule (IV):

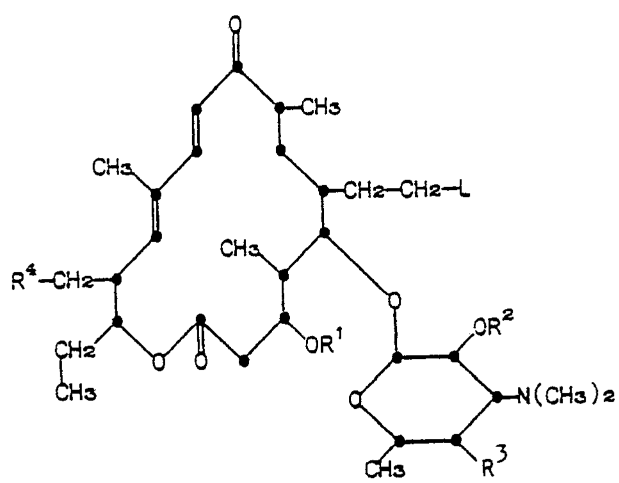

dans laquelle L est un groupe qui s'éloigne et qui est capable d'être déplacé par l'amine HR, dans un solvant organique non réactif, ou

(c) cliver le sucre mycarose d'un macrolide de formule (I) dans laquelle $R^3$ est un groupe mycarosyloxy, par hydrolyse acide pour obtenir un macrolide de formule (I) dans laquelle $R^3$ est un groupe hydroxy, ou

(d) convertir un ester sulfonique de formule (V):

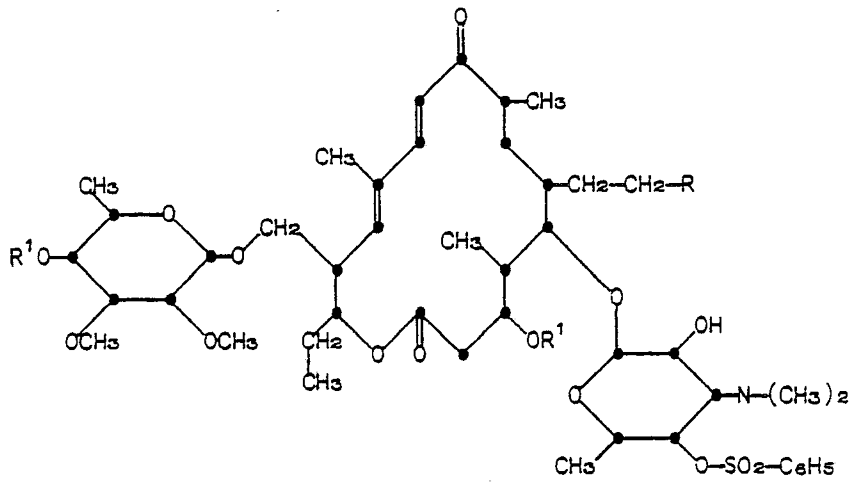

dans laquelle R et $R^1$ ont les significations définies dans la formule (I), en 4'-iodure correspondant en faisant réagir l'ester sulfonique avec une source d'ions iodure dans un solvant organique inerte et, si $R^1$ est différent de l'hydrogène, éventuellement procéder à une hydrolyse pour obtenir le macrolide correspondant dans lequel $R^1$ est un atome d'hydrogène, ou

(e) procéder à une déiodation réductrice d'un macrolide de formule (VI):

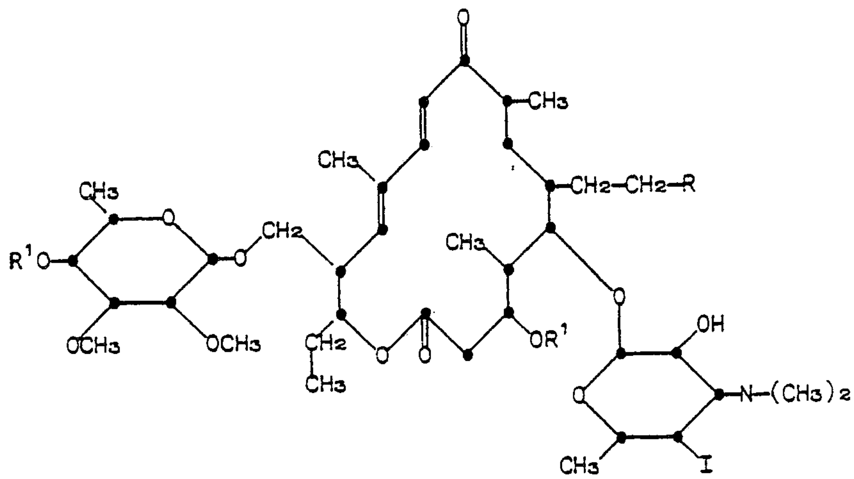

dans laquelle R a la signification définie dans la formule (I) et chaque radical $R^1$ est un groupe protecteur du groupe hydroxy, pour obtenir un macrolide de formule (I) dans laquelle $R^3$ représente H et éventuellement éliminer les groupes protecteurs du groupe hydroxy pour obtenir un macrolide de formule (I) dans laquelle $R^1$ et $R^3$ représentent chacun un atome d'hydrogène, et

(f) au besoin, estérifier et/ou salifier un produit de la réaction (a), (b), (c), (d) ou (e).

8. Macrolide de formule (I) ou un de ses sels pharmaceutiquement acceptables selon l'une quelconque des revendications 1 à 6 en vue de l'utiliser comme antibiotique dans la chimiothérapie des animaux à sang chaud.

9. Prémélange d'aliments comprenant, comme ingrédient actif, un macrolide de formule (I) ou un de ses sels pharmaceutiquement acceptables selon l'une quelconque des revendications 1 à 6.

10. Formulation vétérinaire comprenant, comme ingrédient actif, un macrolide de formule (I) ou un de ses sels pharmaceutiquement acceptables selon l'une quelconque des revendications 1—6, en association avec un ou plusieurs véhicules ou supports physiologiquement acceptables.